# EUROPEAN PATENT SPECIFICATION

(11) **EP 1 503 763 B1**
(45) Date of publication and mention of the grant of the patent: **02.12.2009**
(21) Application number: 03718956.0
(22) Date of filing: 28.04.2003
(51) Int. Cl.: A61K 31/513, A61M 15/08

(54) **THERAPEUTIC 1,2,3,6-TETRAHYDROPYRIMIDINE-2-ONE COMPOSITIONS AND METHODS THEREWITH**
THERAPEUTISCHE ZUSAMMENSETZUNGEN ENTHALTEND 1,2,3,6-TETRAHYDROPYRIMIDIN-2-ONE UND ENTSPRECHENDE METHODEN
COMPOSITIONS THERAPEUTIQUES DE 1,2,3,6-TETRAHYDROPYRIMIDINE-2-ONE ET METHODES ASSOCIEES

(30) Priority: 02.05.2002 US 139193; 08.07.2002 US 191481; 29.08.2002 US 232798; 29.08.2002 US 233126; 08.10.2002 US 267896
(43) Date of publication of application: 09.02.2005
(73) Proprietor: Cragmont Pharmaceuticals, LLC, Berkeley, California 94708 (US)
(72) Inventor: WEI, Edward, T., Berkeley, CA 94708 (US)
(74) Representative: Paget, Hugh Charles Edward
(86) International application number: PCT/GB2003/001811
(87) International publication number: WO 2003/092697

(56) References cited:
- WO-A-99/52520
- GB-A- 1 476 351
- US-A- 3 821 221
- DATABASE CA [Online] CHEMICAL ABSTRACTS SERVICE, COLUMBUS, OHIO, US; WEI, E. T. ET AL: "AG-3-5: a chemical producing sensations of cold" retrieved from STN Database accession no. 99:151925 XP002250384 cited in the application & JOURNAL OF PHARMACY AND PHARMACOLOGY (1983), 35(2), 110-12 ,
- MCKEMY, D. D. ET AL: "Identification of a cold receptor reveals a general role for TRP channels in thermosensation" NATURE., vol. 416, 2002, pages 52-58, XP002250383 MACMILLAN MAGAZINES., US ISSN: 0028-0836 cited in the application

## Description

### BACKGROUND OF THE INVENTION

### Field of the Invention

This invention generally relates to use of a class of chemicals that specifically activate the cold receptors to prepare therapeutic compositions eliciting long-lasting cooling or soothing properties, particularly when formulated for delivery to suppress the sensations of itch and pain, such as for delivery to inflamed skin, to the mucous membranes of the eyes, airways, and anogenital areas, and to the enteric mucosa. The particularly preferred embodiment compositions comprise "icilin", a 1,2,3,6-tetrahydropyrimidine-2-one compound.

### Description of Related Art

Human awareness of the external environment is conveyed by specialized organs for sight, smell, sound, taste, touch and pressure. In addition to these sensory modalities, there are present in the skin, mucous membranes of the eyes, airways, anogenitalia, and gut, specialized nerve endings for detecting heat, cold, pain and itch.

*Anti-inflammatory effects of cold-receptor activation.* The ability of ice applied to the skin or to the eye orbit to suppress the pain and swelling of bums or other forms of traumatic injury is well known. An ice cube in the mouth will also suppress a toothache. The precise mechanisms of pain relief by cold are not clear but it has been shown in animal experiments that the discharge of pain fiber afferents are decreased by contact with surfaces of solids with a low ' temperature such as ice. Menthol, a chemical that activates the cold receptor, does not suppress severe pain, but the cooling sensations that it evokes on the skin and mucous membranes are refreshing and counteract heat and irritation. Thus, menthol is used as a component in a wide range of liniments and lotions for topical application on the skin, and as a flavoring and refreshing agent in foodstuffs, beverages, toothpastes, skin rubs, and mouthwashes. Menthol is also used as a tobacco additive for producing a cool sensation in the mouth and upper respiratory tract when smoking.

*Background on icilin.* 1,2,3,6-Tetrahydropyrimidine-2-one compounds were described in U.S. Patent 3,821,221(inventors C. Podesva and J.M. Do Nascimento., June 28, 1974). These compounds were thought to have depressant and/or stimulant effects on the central nervous system. In 1972, an abstract described a compound in this series called AG-3-5 (1[2-hydroxyphenyl]-4-[3-nitrophenyl]-1,2,3,6-tetrahydropyrimidine-2-one). This prototype elicited a syndrome of "wet dog shake behavior" in rats and monkeys accompanied by hyperthermia, hyperactivity and ptosis. Wei (Chemical stimulants of shaking behavior. Journal of Pharmacy and Pharmacology 28: 722-724, 1976) provided the first detailed report of the actions of AG-3-5 in animals and noted that shaking behavior similar to those of a dog when wet could be evoked in various laboratory animals such as the rat, mouse, cat, dog, gerbils, guinea pigs and hamsters. Subsequently, Wei (Pharmacological aspects of shaking behavior produced by AG-3-5, TRH, and morphine withdrawal. Federation Proceedings 40: 1491-1496, 1981) reported that 0.1 mg of AG-3-5 applied to the dorsum of the tongue elicited prickling sensations of cold and ingestion of 6 mg, on one occasion out of three, produced sensations of coolness on the cheeks and on the inner surfaces of the arms and legs. It was hypothesized that AG-3-5 may produce specific activation of receptors for cold, and that stimulation of these receptors accounted for the shaking seen in laboratory animals. In a subsequent publication (B.T. Wei and D.A. Seid. AG-3-5: A chemical producing sensations of cold. Journal of Pharmacy and Pharmacology 35: 110-112, 1983) the effects of AG-3-5 on shaking behavior in the rat were compared to those of menthol and AG-3-5 was shown to be 400 times more potent than menthol on a molar basis on this behavioral endpoint. AG-3-5 was less toxic than menthol, as measured by the oral median lethal dose in rats. AG-3-5 was named icilin because of its cold-producing properties.

Recently, the molecular bases of thermosensation have been elucidated. Two groups cloned a biological macromolecule (called receptor) from trigeminal sensory neurons of the rat that responds to cold temperatures, menthol and icilin. These receptors belong to the transient receptor potential (TRP) family of ion channels and are named TRP-M8 receptors. Using a sample provided by Wei, McKemy et al. (Identification of a cold receptor reveals a general role for TRP channels in thermosensation. Nature 416: 52-58, 2002) showed that icilin was about 200 times more potent than menthol in eliciting ion channel current changes in the cloned and transfected TRP(M8) receptor. The ion permeability changes elicited in transfected cells were more robust with icilin than those elicited by menthol, and the presence of extracellular calcium was required for activity. Menthol currents did not require extracellular calcium.

The chemical structure of icilin bears little similarity to that of menthol; the former one being a pyrmidine-2-one attached to two phenyl rings, and the latter a cyclohexanol derivative. Activation of the TRP-M8 receptor on the neuronal membrane may lead to depolarization of the sensory nerve ending and send action potentials towards the spinal cord and brain that are eventually recognized as psychic signals of cold. Chemical agents that activate the TRP-M8 receptors are called TRP-M8 receptor agonists.

A need exists for therapeutic compositions useful on inflamed skin and the mucous membranes of the body for the relief of pain and itching that accompanies inflammation.

### BRIEF SUMMARY OF THE INVENTION

The invention consists in the use of a cold receptor agonist for the manufacture of a topical therapeutic composition, as set out in claim 1. The therapeutic composition preferably further comprises one or more pharmaceutically active drugs such as an anti-inflammatory glucocorticosteroid, a sympathomimetic amine vasoconstrictor or decongestant, an antihistamine, a local anesthetic, menthol or a menthol analog, an immunosuppressant or a carminative. The cold receptor agonist may be represented by the general formula 1-[R1-phenyl]-4-[R2-phenyl]-1,2,3,6-tetrahydropyrimidine-2-one wherein: R1 is -hydroxy, -chloro, - fluoro, -alkyl (with about 2 to 4 carbons), -acetoxy, -trifluoromethyl ; and R2 is -nitro, -chloro, -fluoro, -alkyl, -trifluoromethyl.

A particularly preferred cold receptor agonist embodiment is called "icilin" and a particularly preferred composition is formulated and administered as a lotion, cream or ointment, and preferably in combination with one or more pharmaceutically active drugs, to offer improved therapeutic benefit for the treatment of the pain, itch, and discomforts of inflammation on skin and mucous membranes.

Preferred uses of the compositions are for application to the skin or mucosa, by oral ingestion or inhalation. Thus, in another aspect of the invention, icilin or an icilin analog is formulated for aerosolized delivery to the airways in the treatment of disorders of the airways. In yet another aspect of the invention, icilin or an icilin analog is formulated for localized delivery to the colon in the treatment of irritable bowel.

Other advantages and aspects of the present invention will be understood by reading the following detailed description and the accompanying claims.

### DETAILED DESCRIPITON OF THE INVENTION

Compositions manufactured in accordance with this invention utilize a 1-R1-phenyl, 4-R2-phenyl substituted 1,2,3,6-tetrahydropyrimidine-2-one cold receptor agonist, preferably of the general formula 1-[R1-phenyl]-4-[R2-phenyl]-1,2,3,6-tetrahydropyrimidine-2-one wherein: R1 preferably is -hydroxy, -chloro, - fluoro, -alkyl (with about 2 to about 4 carbon atoms) -acetoxy, -trifluoromethyl ; and R2 preferably is -nitro, chloro, -fluoro, -alkyl (with about 2 to 4 carbons), -trifluoromethyl. I refer to the particularly preferred compound and its analogs as "icilin" and its analogs as "icilin analogs". Formula 1 illustrates the general formula and Formula 2 represents icilin.

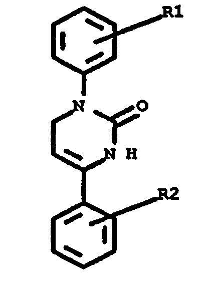

### Formula. 1: 1-R1, 4-R2, substituted 1,2,3,6-tetrahydropyrimidine-2-one

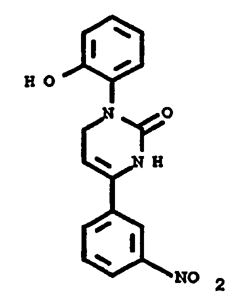

Formula 2: Icilin or 1-(2'-hydroxyphenyl)-4-)3"-nitrophenyl)-1,2,3,6-tetrahydropyrimidine-2-one [Synonyms: AG-3-5, 3,6-Dihydro-1-(2-hydroxyphenyl)-4-(3-nitrophenyl)-2(1H)-pyrimidinone, 1-[2-hydroxyphenyl]-4-[3-nirophenyl]-1,2,3,6-terahydropyrimidine-2-one, 3-(2-hydroxyphenyl)-6-(3-nitro-phenyl)-3,4,dihydro-1H-pyrimidin-2-one].

Icilin is a lemon yellow crystalline powder with a molecular weight of 311.3 Daltons and a melting point of 229 to 231°C. The powder is without odor and non-irritating, meaning that it does not elicit any smell or unpleasant sensations upon contact with the surfaces of the human body. The compound is stable at room temperature. Icilin is readily soluble in organic solvents such as dimethylsulfoxide, nitromethane, dimethylacetamide and propanediols; slightly soluble in ethanol and acetone; and virtually insoluble in water. Thus, icilin would be considered as a lipophilic, hydrophobic compound that is not easily miscible with aqueous systems. Analogs of icilin, for example, with acetyoxy, methoxy, ethoxy, fluoro, or trifluoromethyl substitutions, retain similar chemical and physical properties and are included within the scope of this invention.

Methods suitable for the preparation of the Formula 1 and Formula 2 compounds are described by Podesva and Do Nascimento, U.S. Patent 3,821,221, issued June 28, 1974, incorporated herein by reference, and are exemplified by Example A hereinafter.

Menthol, by contrast, is a white crystal with a molecular weight of 156 Daltons and a melting point of 41 to 43°C, a temperature slightly above body temperature. Menthol has a characteristic peppermint odor and in high concentrations is irritating to the eyes and elicits taste sensations on the tongue and oropharynx. Menthol is more hydrophilic than icilin being soluble in ethanol and slightly soluble in water. The physico-chemical differences between icilin and menthol confer unique advantages to icilin for manipulating sensory nerve discharges in certain defined clinical situations, as described hereinafter. The first advantage is the site of delivery: Menthol cannot be delivered to the eyelids and its environs, the nasal membranes, or the mucous membranes of the anogenital region at high concentrations because it is pungent, stings and irritates. Icilin, on the other hand, can be administered to these sites because it is non-irritating. A second major advantage of icilin is its duration of action. Icilin's actions can be localized at its site of application for a prolonged period because of its lipophilic characteristics and because it can be administered as a powder or dispersed (either in solid form or solubilized) as an emulsion. By contrast, menthol is volatile and rapidly re-distributes and metabolizes in the body so its duration of action is relatively short. For example, icilin applied to nasal membranes can produce refreshing sensations for up to 8 hours. By contrast, menthol applied to the skin or mucous membranes generally does not produce cooling sensations for longer than 15 to 30 minutes, regardless of dose.

As described herein, icilin is shown to be useful as an active antipruritic agent on the skin, and on the mucous membranes of the ocular and anogenital regions. Also, icilin, formulated as an eye-drop, relieves eye irritation and provides refreshing sensations. Yet further, icilin may be encapsulated and administered to relieve irritation in the gut mucosa. One major conclusion in practicing this invention is that icilin, applied to inflamed skin and mucous membranes, will relieve itch, irritation and pain.

Icilin is particularly contemplated for use when it is combined (used as an adjuvant) with other drugs such as anti-inflammatory glucocorticosteroids, sympathomimetic amine decongestants, antihistamines, local anesthetics, menthol and menthol analogs, immunosuppressants, or a carminative such as peppermint oil. Icilin administered in combination with such drugs offers greater therapeutic benefit than formulations that do not include icilin. Table 1 summarizes the use of icilin formulations to relieve itch, irritation, and pain in various inflamed tissues and examples of medical conditions associated with the inflammation. Table 1. Summary of Uses of Icilin Formulations for Various Inflammations.

| **Icilin Formulations** | **Target Tissues** | **Medical Conditions** |
|---|---|---|
| liquid (eye drops) or ointment | eyelids and eyeball surfaces-mucocutaneous | pain after corneal abrasions, laser surgery, orbital and eyelid, and eye surface inflammation |
| spray, lotion, cream or ointment | inflamed skin | itch, irritation, pain, atopic dermatitis, eczema, psoriasis, insect bites, infections, bums |
| liquid spray, powder | nasal epithelium, mucocutaneous | nasal stuffiness from the "common cold", rhinitis, nasal irritation and congestion |
| inhaled powder | nasal epithelium, mucous membranes of the pharynx and bronchus | snoring and sleep apnea, dyspnea, breathing disorders |
| liquid spray, inhaled powder | mucous membranes of the pharynx and bronchus, mucous membranes | bronchial constriction and congestion from asthma, chronic obstructive pulmonary disease |
| ointment, lozenge | mouth, oropharynx, mucous membranes | pain from mucositis, e.g. after chemotherapy for cancer, oral cavity sores |
| lozenge, chewing gum | esophagus, stomach, duodenal mucosa | esophagitis, gastrointestinal esophageal reflux disease (GERD), peptic ulcer |
| lipstick, ointment, cream | lips-mucocutaneous | angular cheilitis, cheilitis, herpes simplex infection, mouth and lip sores |
| oral formulation | enteric and colonic mucosa | irritable bowel syndrome |
| oral formulation | colonic mucosa | colitis |
| ointment-cream | anus-mucocutaneous | anal fissure |
| ointment-cream | perianal skin, anus | hemorrhoids, perineal, "diaper rash", inflammation, pruritus ani |
| suppository, enema | rectal mucosa | proctitis (e.g. caused by radiation or ulcerative colitis), hemorrhoids |
| suppository, enema | anus, rectal mucosa | anorectal discomfort |
| lotion-cream-ointment | anus / penis - mucocutaneous | sexual dysfunction-homosexual |
| lotion-creamointment | penis-mucocutaneous | sexual dysfunction-heterosexual |
| lotion-creamointment | vaginal, clitoral surfaces, mucocutaneous | sexual dysfunction, vulvar itch, vaginitis, vaginal allodynia, dyspareunia |

### General Formulation of Icilin Compounds for Delivery to the Cold Receptor

The formulations of icilin compounds (icilin and/or its analogs) for delivery to cold receptors depend on the accessibility in various tissue beds and the desired duration of action. Standard lotion, cream, and ointment formulations may be used for delivery to inflamed skin and to the mucous membranes of the lips, eyes and anogenital areas. Thus, particularly preferred embodiments of this invention are wherein icilin is used as an adjuvant so as to improve the efficacy of the lotion, cream or ointment because it reduces the patient's sense of discomfort and itch and alleviates some aspects of the inflammation. The surprisingly long duration of action of icilin greatly expands the efficacy of treatment.

For delivery to cold receptors lining the nasal cavity, icilin dissolved first in an inert solvent such as propanediol and then suspended in aqueous saline, can be delivered with a nebulizer. In such a device, commonly used for delivering a decongestant in a nasal spray, liquid droplets, of a size pattern not likely to penetrate the lower respiratory tract, are generated by manual pressure on a squeeze bottle with a defined nozzle size. The drug effects are then localized to the nasal cavity.

For delivery to the cold receptors located in the mucous membranes of the upper airways (bronchi and bronchioles), icilin may be delivered is soluble form with standard metered dose inhalers or as a crystalline material in dry powder inhalers. Both methods of delivery allow precise control of dosage and location of drug delivery.

For delivery to cold receptors in the skin or mucous membranes, the physical features of the icilin molecule may be modified to confer greater accessibility to the target. For example, the drug may be re-crystallized or micronized to a smaller particle diameter and/or greater surface area to facilitate solubility in tissues. Standard skin permeability enhancers such as surfactants may be used for increasing penetration to receptors in the skin, and a standard drug carrier such as petrolatum or liposomes may be used for drug delivery.

Yet further, icilin may be applied as a liniment or encapsulated in and/or bound to resins with chemical bonds that are selectively cleaved in the intestinal tract to access cold receptors in enteric tissues. Further description of such embodiments is given below.

### Treatment of Pruritus

The skin is composed of three layers: the epidermis, the dermis and the subcutaneous fatty tissue. The epidermis varies in thickness from 0.15 to 0.80 mm and its outermost part, called the stratum corneum (horny layer), is built of several layers of flattened, dehydrated, keratinised cells. The dermis is 3 to 5 mm thick and it contains collagen fibers, blood and lymphatic vessels, hair follicles, sebaceous and sweat glands and the drug target - the sensory nerve endings.

Itch is a familiar sensory state associated with the desire to scratch. Itching and scratching are phenomena common to humans and animals. The sensory nerves that initiate itch have not been precisely identified but are thought to be unmyelinated C-fibers, with nerve endings located between the epidermis and dermis. In medical terminology, severe itching is called pruritus and drugs that suppress itching are anti-pruritic agents. Many inflammatory and irritating conditions of the body are expressed as itching together with pain. Itching occurs mainly on skin, on mucous membranes, and on the conjunctiva. One aspect of this invention is to relieve itch with an anti-pruritic composition.

Pruritus of the skin may be caused by dermatoses such as contact dermatitis, atopic dermatitis, psoriasis, seborrheic dermatitis, autosensitization dermatitis, asteatosis, senile pruritus, photosensitive dermatosis, urticaria, prurigo, impetigo, eczema, lichen, sunburn, and acne vulgaris. Included in such dermatoses is also itching caused by poison oak, and allergies to plants such as oak and sumac. Pruritus of the skin may also be caused by infectious agents such as herpes virus (for example, itchy cold sores from herpes simplex virus), insects and parasites. For example, scabies is caused by a tiny mite (Sarcoptes scabiei). The mites burrow into the skin and cause itching. The mites may be removed by drugs such as permethrin but the residual mite fragments can initiate an allergic state leading to severe itching. Pediculosis is an infestation of the hairy parts of the body or clothing with the eggs, larvae or adults of lice. The crawling stages of this insect feed on human blood, which can result in severe itching. Head lice are usually located on the scalp, crab lice in the pubic area and body lice along seams of clothing. Problems with lice infestations afflict million of Americans, particularly school children. Enterobiasis (pinworm) is caused by a small, white intestinal worm called Enterobius vermicularis. Pinworms are about the length of a staple and live in the rectum of humans. While an infected person sleeps, female pinworms leave the intestines through the anus and deposit eggs on the surrounding skin. Itching around the anus, disturbed sleep, and irritability are common symptoms. Fungal infections of the skin, for example, athlete's foot, and infections of the anogenital region, for example, yeast infections in women, are also conditions that cause pruritus.

Blepharitis and conjunctivitis are caused by inflammation of the membranes of the ocular orbit and eyeball, and may lead to severe itching. Examples of visceral diseases complicated with pruritus and being particular problems include malignant tumors, diabetes mellitus, hepatic diseases (especially cholestasis), renal failure, hemodialysis, and pregnancy.

The anorectal region is a frequent location for inflammation and itch. Hemorrhoids, perineal inflammation, and diaper rash are causes of itch, as well as soiling of the skin from diarrhea, such as may occur in irritable bowel disease. Enterobiasis, as described above, is another cause of itching in this region.

Surprisingly, powerful topical anti-pruritic agents that target pathological itch are not currently described in the pharmacological literature. Drug therapies that reduce itching include drugs such as glucocorticosteroids, sedative antihistamines, and some tricyclic compounds, such as doxepin hydrochloride, but these lack efficacy for severe itch or, upon continued use, have undesirable side effects. Topical anti-inflammatory glucocorticoids are widely used for dermatoses and have short-term effectiveness for reducing inflammation. Prolonged use, however, results in thinning of the skin and the possibility of super-infection. Vitamin D compositions have been described as possessing anti-pruritic properties.

The significance of itching in clinical conditions is illustrated by the condition known as atopic dermatitis (also known as atopic eczema). Atopic dermatitis is a chronic inflammatory skin disorder exhibited by individuals with a hereditary predisposition to a lowered cutaneous threshold to pruritis, often accompanied by allergic rhinitis, hay fever, and asthma. The condition is characterized by extreme itching, leading to scratching and rubbing that in turn results in the typical lesions of eczema. In infants (infantile eczema), there is a predilection for occurrence of itch on the cheeks, which may extend to other areas of the body. In older children, adolescents and adults, it is found chiefly on the flexural surfaces, especially on the antecubital (elbow) and popliteal (knee) areas, and on the neck, eyelids, and wrists and behind the ears.

Affected children will scratch even when asleep for up to 2 out of 8 hours of sleep (versus a few minutes for normal children) and the excoriations can lead to bleeding and infection. Atopic dermatitis and eczema, if sufficiently severe, can lead to death. Less serious, but uncomfortable and often painful symptoms associated with atopic dermatitis include swelling, redness, blisters, crusting, ulceration, pain, scaling, skin cracking, hair loss, scarring, or oozing of fluid involving the skin, eye, or mucous membranes. Under current therapy, topical glucocorticosteroids are only partially effective in treating this condition. Newer immunosuppressant drugs such as Elidel® and Protopic® suppress the skin inflammation in individuals with atopic dermatitis, but the application of these drugs causes skin irritation.

Compositions of the present invention with icilin and/or icilin analogs are preferably formulated, as liniments with a dermatologically acceptable vehicle in which icilin and/or icilin analogs are dispersed, and may preferably further comprise a single pharmaceutical or a combination of pharmaceuticals. Pharmaceuticals that may be used, either alone or in combination, include anti-inflammatory analgesic agents, anti-inflammatory steroidal agents, antihistamines, sympathomimetic amine vasoconstrictors, menthol and menthol analogs, local anesthetics, immunosuppressants, anti-fungal agents, antibiotics and keratolytics.

Examples of anti-inflammatory analgesic agents include methyl salicylate, monoglycol salicylate, aspirin, indomethacin, diclofenac, ibuprofen, ketoprofen, naproxen, pranoprofen, fenoprofen, sulindac, fenclofenac, clidanac, flurbiprofen, fentiazac, bufexamac, piroxicam, pentazocine, and the like.

Examples of steroidal anti-inflammatory agents include hydrocortisone, prednisolone, dexamethasone, triamcinolone acetonide, fluocinolone acetonide, hydrocortisone acetate, prednisolone acetate, methylprednisolone, dexamethasone acetate, betamethasone, betamethasone valerate, flumetasone, fluticasone, fluorometholone, beclomethasone diproprionate, and the like.

Examples of antihistamines include diphenhydramine hydrochloride, diphenhydramine salicylate, diphenhydramine, chlorpheniramine maleate, promethazine hydrochloride, Patanol® and the like.

Examples of sympathomimetic amine vasoconstrictors include phenylephrine hydrochloride and napthoxymetazoline, oxymetazoline, and the like.

Examples of menthol analogs include WS-23, WS158 and other phosphine oxide menthol analogs, menthoxypropane-1,2-diol and cyclic alpha-keto enamines such as 4-methyl-3-(1-pyrrolidinyl)-2-[5H]-furanone.

Examples of local anesthetics include dibucaine hydrochloride, dibucaine, lidocaine hydrochloride, lidocaine, benzocaine, pramoxine hydrochloride, tetracaine, tetracaine hydrochloride, oxyprocaine hydrochloride, mepivacaine, piperocaine hydrochloride, and the like.

Examples of antibiotics include neomycin and the anti-viral agent docosanol (Abreva®), and examples of keratolytics include such agents as, alpha-hydroxy acids, glycolic acid and salicylic acid.

Examples of smooth muscle relaxants include nitric oxide donors, such as minoxidil, isorbide dinitrate, and phosphodiesterase inhibitors, such as aminophylline. Other examples of smooth muscle relaxants that may be given in combination with icilin to achieve relaxation of the internal anal sphincter in anal fissures are described in U.S. Patent 6,391,869.

Examples of menthol analogs include WS-3 and WS-23, both menthol analogs having Generally Recognized As Safe status by regulatory authorities; WS-158 and other phosphine oxide menthol analogs; menthone glycerol ketal, menthyl lactate, menthoxypropane-1,2-diol and cyclic alpha-keto enamines such as 4-methyl-3-(1-pyrrolidinyl)-2-[5H]-furanone. A preferred menthol analog is WS-158, a phosphine oxide menthol analog synthesized by Wilkinson Sword Company [Watson, H. R., R. Hems, D.G. Roswell & D. J. Spring, New compounds with the menthol cooling effect, J. Society Cosmetic Chemists. 29, 185-200 (1978)]. The duration of the cold effect for this combination lasts for more than 4 hours whereas normal menthol analogs alone seldom act longer than 1 hour. Such a medication is useful for human therapeutics as well as for veterinarian uses.

Examples of immunosuppressants are mycophenolic acid, including its salt form known as mycophenolate mofetil (Cellcept®), tacrolimus (Protopic®) and pimecrolimus (Elidel®), both of which are topical immunosuppressive drugs used in the treatment of atopic dermatitis and eczema.

### Treatment of Pain and Inflammation of the Skin and Mucous Membranes

The skin of the lips and anogenital region, and surrounding tissues, are densely supplied with nerve fibers and these afferent (A delta and C) fibers code for pain and temperature signals. When the lips, anus, genitalia, or the tissues surrounding, are injured, the subsequent inflammation stimulates the sensory nerve endings and the result is pain and sometimes itch. The mucous membrane of the inner lips, mouth, and rectum also contain nerve endings that transmit signals of pain, but these signals are less discrete and are also sensitive to stretch. For example, excessive stimulation of rectal nerve endings produces a sense of distension, an urge to have a bowel movement, flatulence, and, if the stimulation is excessive, rectal pain and discomfort. *Inflammatory Conditions of the Lips and Mouth.* The skin and mucosa of the lips are demarcated by the vermilion border. The mucosa seen on viewing the face is keratinized and dry; the mucosa of the inner aspect of the lips is nonkeratinized and moist. Branches of the 5th cranial nerve (trigeminal) innervate these tissues. Non-specific disorders can cause inflammation around the mouth and lips. These include immunological disorders, such as pemphigus, pemphigoid, lichen planus; infections, such as venereally transmitted warts and herpes simplex; metaplasia, such as dyskeratosis; and physical causes, such as lip and mouth surgery, sunlight, and wind. Some common conditions affecting the surface of the mouth and lips are:
*Angular Cheilitis*-Chelitis is the technical term for inflammation of the lips. Angular cheilitis occurs at the corners of the lips. This lesion is seen as dry, scaly, red skin and there may be a fissure or slit in the mucocutaneous junction, causing severe pain when the mouth is opened wide. Angular cheilits may be caused by dry weather, too much sunlight, allergic reactions, or excessive salivation. Cheilitis is also seen more frequently in persons who have reduced immune function, viral infections, or persistent yeast infection. Other forms of inflammation of the lips are cracking exfoliative cheilitis, cheilitis glandularis, and cheilitis granulomatosa.
*"Cold sores"* or herpes labialis, are the result of an infection with a common virus known as herpes simplex. The sores begin as a group of small red bumps that blister. Itching and burning of the area precedes the prodroma phase (the time for onset of sores). The blisters begin to dry up after a few days and form a yellow crust. The crust then falls off and the redness slowly goes away. The whole process takes about 10 to 14 days. The sores frequently occur on the vermilion border and on the corners of the lips. Infections are more common in immunodeficient individuals, and the blisters themselves are infectious.
*Stomatits,* or inflammation of the inner mouth, can occur after infections, or when cancer chemotherapeutic drugs are administered (a condition that is also called mucositis), or when the head and neck are irradiated for cancer treatment.
*Inflammatory Conditions of the Anorectal Area.* The area between the anus and the genitalia is called the perineum. The anus is the orifice at the terminus of the intestinal tract. Just behind the anus is the anal canal wherein lies the exterior sphincter and the interior sphincter. A sphincter is a circular muscle that constricts and relaxes. Anal sphincters constrict to retain feces and expand to allow it and flatus to pass during defecation. The anal canal is the first 2 inches of skin after the anus; it is closed while at rest and open during defecation. It leads to the rectum, the cavity that runs vertically from the end of the colon to the anal canal. The rectum is approximately 5 inches long and 1.5 inches wide. It stores feces prior to defecation. The term "anorectal area" is defined herein to include the perineum, anus and rectum of a human. More particularly, the term includes the external anus, the internal anus and the lower rectum.
The nerve supply of the anorectal area controls its sensory and motor functions. The inferior rectal nerve, a branch of the pudendal nerve, innervates the lower portion of the anal canal and the external sphincter. These tissues have somatosensory nerve endings, responding to pain, touch and temperature. By contrast, the hypogastric nerve (a visceral nerve because it is part of the autonomic nervous system) innervates the upper portion of the anal canal, internal sphincter and rectum. The sensory nerve endings of the hypogastric nerve respond to distension and transmit visceral afferent signals, including pressure, pain and temperature, to the spinal cord, but discrete sensations are not localized. Instead, over-stimulation of the visceral sensory nerve endings evokes a sense of bloat, distension, cramping, and an urge to defecate.
Many agents or conditions can cause injury to the tissues of the anorectal area and produce pain and inflammation. The primary symptoms are pain and itch but if the inflammation extends to the lower bowel mucosa (rectum, colon, ileum) and its nerve endings, there are also distressing sensations of visceral discomfort. Some of the pathological conditions are described below.
*Perineal Dermatitis.* Inflammation of perineal skin occurs when urine or feces, because of incontinence, irritates the skin. Pads (e.g. diapers) or other containment devices for incontinence may exacerbate inflammation by causing perspiration and prolonging contact of irritants with tissues. The dermatitis that results causes itch, pain, infections and may lead to ulceration of the skin. Treatment using cleansers, moisturizers, moisture barriers -including ointments, creams, powders, pastes, and lipid barrier films, are discussed by Gray et al. (Perineal Skin Care for the Incontinent Patient. Advances in Skin and Woundcare 15: 170-177, 2002), incorporated herein by reference. An ointment, powder, aerosol or paste containing icilin will alleviate pain and itch of such conditions in the elderly and in the infant (e.g. diaper rash) and facilitate therapeutic management.
*Anal Fissure.* A fissure is a break or slit in tissue usually at the junction of skin and mucous membrane (mucocutaneous junction). If the fissure occurs on the corner of the mouth, it is called angular cheilitis and opening the mouth can cause pain. An anal fissure is a small tear in the skin of the anus. The fissure can bleed, itch, and become extremely sore during and after defecation, making bowel movements, sitting, and sex painful. Anal sex without lubrication, a hard bowel movement, and inflammation of anal tissue can cause a fissure. Some heal within a couple of weeks, especially if therapeutic cream, warm baths, and stool softeners are used. Others require surgical correction of the tear but this is associated with a subsequent risk of incontinence. Ointments for the treatment of anal fissure have been described in a patent from Cellegy U.S Patent 6,391,869, incorporated herein by reference. These ointments contain, as active ingredients, substances that release nitric oxide and increase cyclic AMP of smooth muscle cells in the internal anal sphincter. The nitric oxide relaxes the sphincter smooth muscle and the reduced tension decreases pain signals from the fissure.
*Hemorrhoids.* Hemorrhoids are swollen veins that may protrude outside the anus or reside inside the anal canal. The veins may become inflamed, blocked, or broken and cause fecal contamination of perianal skin. Hence, hemorrhoids may bleed and cause pain and itching because of incontinence and excessive wiping (pruritus ani). Risk factors for hemorrhoids include strenuous or frequent bowel movements that raise intra-abdominal pressure and impede venous return. Related risk factors for developing hemorrhoids are birth trauma and lifting weights.
*Sexually Transmitted Diseases.* A number of sexually transmitted diseases (STD) can have local effects on tissues in the anorectal and genital region, producing pain, itch, redness and blisters. These STD include: Herpes simplex virus (HSV), an infection of the skin and genital mucosa (mucous membrane) causing recurring sores and pain, Gonorrhea, an inflammation of genital mucosa, causing infectious and painful discharge, and Human papillomavirus (HPV) causing genital and anal warts
*Proctitis* is inflammation of the lining of the rectum. Proctitis can be short term (acute) or long term (chronic). Proctitis has many causes. It may be a side effect of autoimmune diseases of the lower bowel, such as ulcerative colitis and Crohn's disease. Sexually transmitted diseases may cause proctitis. Proctitis is frequently a side-effect of radiation used to treat prostate cancer or cancer of the female organs. The rectum resides just behind the prostate in the male and the vagina and uterus in the female, so when these organs are irradiated, the bowel wall is injured. Radiation proctitis is manifested as the new growth of many tiny blood vessels on the epithelial surface of the rectum. These blood vessels are fragile and bleed with minimal trauma, resulting in blood in the stool. If the bleeding is severe, anemia or a low red cell blood count can occur. Other causes of proctitis include traumatic rectal injury, allergies, and malfunction of the nerves in the rectum. Pathogenic organisms that may cause anorectal inflammation are *Salmonella, Shigella, Campylobacter, Entamoeba histolytica, Giardia lamblia, Mycobacterium avium-intracellulare, Cryptosporidium, Microsporidium,* and *Cytomegaolvirus.* Patients infected with human immunodeficiency virus or receiving immunosuppressive drugs are especially susceptible to infectious agents attacking the lower gut lining and surrounding tissues.
The most common manifestation of proctitis is a frequent or continuous sensation of an urge to have a bowel movement. This distressing but ineffectual urge to empty the rectum is called tenesmus. Other symptoms include constipation, a feeling of rectal fullness, left-sided abdominal pain, passage of mucus through the rectum, rectal bleeding, and anorectal pain. Proctitis because it leads to bleeding, some incontinence, and inflammation will produce subjective discomfort, pain, and itch in the entire anorectal area.
*Inflammatory Conditions of the Genitalia.* The male and female genitalia are covered with skin and mucous membranes that are densely innervated by sensory fibers for pain, itch, and thermosensation. These issues may be inflamed by physical agents, such as excessive friction, by chemical agents such as topical drugs, and by biological agents such as infectious fungi. Vulvovaginitis account for over 10 million once visits per year. The most common infectious organism is *Candida Albicans* and the over-the-counter sales of anti-fungal remedies is over $250 million per year. Other recurrent problems of female genitalia are vaginal vestibulitis, vaginal alloydynia, dyspareunia, and irritant dermatitis. A topical agent that relieves the itch, pain, and irritation caused by vulvovaginits would have considerable utility.

### Treatment of Eyelids and Eye Surface

The irritants that generate itch in the skin, sneezing in the nasal cavity and cough in the upper airways also irritate the eyelids and eye surfaces. The eyeball is a dynamic system of fluid secretion and sensory nerve endings. Minor irritation evokes blinking and lachrymation. It is known to this art that certain patients suffer from ocular and/or palpebral sensations of itching or pruritus and dysesthesic sensations around the eyes and eyelids. These pruritic or dysesthesic sensations may be of allergic origin, of infection, or of tissue injury. By the term "dysesthesic sensations" are intended sensations of burning or heating, stinging, tingling, strain, discomfort and tightness. These sensations may be combined with redness. Together, these conditions are also called "pink eye" or conjunctivitis. Among the factors triggering ophthalmic or palpebral pruritic or dysesthesic afflictions, exemplary thereof are rapid temperature variations, heat and in particular exposure to ultraviolet or infrared radiation, low relative humidity, exposure to violent winds or to currents of air (blowing machine, conditioned air), the application of surfactants, exposure to toxic or irritant vapors or to dusts, irritant topical products, irritant dermatological or cosmetic palpebral topical products or the use of certain cosmetics, even when these are not known to be particularly irritating. Other factors triggering ocular or palpebral pruritic or dysesthesic afflictions which should also be included are allergens such as, in particular, pollen, animal hairs, acarians and molds, and mechanical injuries such as occur during corneal abrasions or during corneal transplants, and other ocular inflammatory conditions of genetic or immunological disorders. The pathological mechanism of these signs are poorly understood and ocular and/or palpebral dysesthesias are treated with corticosteroids and also local antiseptics as an ophthalmic ointment or as drops. Although corticosteroids are relatively effective at alleviating the above symptoms, they have, however, side effects that are often severe, such as atrophies and super-infections.

Thus, prior art treatments of the aforesaid ocular and palpebral dysesthesias, pains and pruritus have significant drawbacks and disadvantages, which are overcome by practice of the present invention.

An icilin eye-drop preparation to reduce pain, itch, irritation and to provide "refreshment" to eye sensations may consist of each milliliter of buffered ophthalmic solution in a suitable ophthalmic dispenser containing icilin, dissolved in an approved non-irritating solvent such as 1,2-propanediol, equivalent to 0.1 to 5 mg/ml (%) icilin, and inactive ingredients: creatinine, sodium citrate, sodium borate, polysorbate 80, disodium edetate, adjusted pH to 6.6 -7.2, and water. Benzalkonium chloride 0.02% and sodium bisulfite 0.1% are added as preservatives. This solution may also contain adjunct drugs such aqueous soluble steroids (e.g. dexamethasone sodium phosphate) and antibiotics (e.g. neomycin). To prolong duration of action on the eyelids and eye surface, icilin may also be administered as a micronized particle suspended in solution, admixed with an inert carrier, or with an inert binding substance, e.g. alginate. An example of an ophthalmic solution to which icilin may be added is Neodecadron®. Each milliliter of buffered Ophthalmic Solution NEODECADRON in the OCUMETER ophthalmic dispenser contains: dexamethasone sodium phosphate equivalent to 1 mg (0.1%) dexamethasone phosphate, and neomycin sulfate equivalent to 3.5 mg neomycin base. Inactive ingredients: creatinine, sodium citrate, sodium borate, polysorbate 80, disodium edetate, hydrochloric acid to adjust pH to 6.6 - 7.2, and water for injection. Benzalkonium chloride 0.02% and sodium bisulfite 0.1% are added as preservatives.

### Treatment of Nasal Irritation, Stuffiness, and Congestion

The nose is the entrance to the respiratory tract. It serves as a conduit for inspired and expired air. When one or both sides of the nose are obstructed, this impairs nasal functioning and is perceived as an uncomfortable condition. All the nasal cavity bony surfaces, including the paranasal sinuses, are lined by tissue called mucosa. This mucosa contains blood vessels, nerves, and small glands that secrete fluids into the nasal cavity. The nose is supplied by nerves from the trigeminal branch, which are capable of detecting pain, temperature and pressure. The nasal mucosa functions to humidify and warm the inspired hair, hence it receives a large blood flow and the cells maintain a high degree of metabolic activity. Inflammation of the nasal mucosa caused by allergy, irritants or infections will cause the mucosa to swell. When the mucosa swells, the area available through which air can pass is diminished, and therefore one experiences a sense of nasal obstruction. The nose can also become "runny" (rhinitis) and the fluid discharge adds to the feeling of congestion. If either or both sides of the nose are obstructed, the asymmetry in airflow is perceived as an unpleasant condition.

Icilin administered into the nasal cavity, as a spray or powder, produces a sensation of increased airflow, refreshment and coolness, without any odor or sense of irritation. The mechanism of this effect may be because the nasal mucosa is lined with vanilloid (capsaicin receptor 1, VR1) and cold receptors. The vanilloid receptor is linked to sensory nerve afferents that are activated by painful stimuli. Stimulation of these nerve fibers also lead to antidromic release of vasodilatory substances and inflammatory mediators such as histamine, substance P and calcitonin-gene related peptides, a process called neurogenic inflammation. Babes et al. [Cooling inhibits capsaicin-induced currents in cultured rat dorsal root ganglion neurones. Neurosci Lett 317: 131-134,2002] have shown that reducing the temperature in dorsal root ganglion neurones inhibited discharge of the vanilloid receptor. The soothing and decongestant actions of icilin may be explained by such results on cold and vanilloid receptor interactions. Icilin stimulation of receptors may mimic the cold inhibition of vanilloid receptor activity and hence reduce the inflammation occurring in the upper airways.

Nasal stuffiness and congestion has many causes, the most common being "rhinitis", a technical term meaning the condition of inflammation of the membranes lining the nose. Rhinitis is characterized by nasal congestion, rhinorrhea ("runny nose"), sneezing, itching of the nose and/or postnasal drainage. A common form of rhinitis is seasonal allergic rhinitis which is caused by an immunoglobulin E (IgE)-mediated reaction to seasonal aeroallergens. Typical seasonal aeroallergens are pollens and molds. The length of seasonal exposure to these allergens is dependent on geographic location. Perennial allergic rhinitis is caused by an IgE-mediated reaction to perennial environmental aeroallergens. These may include dust mites, molds, animal allergens, or certain occupational allergens, as well as pollen in areas where pollen is prevalent perennially. Allergic rhinitis frequently coexists with allergic conjunctivitis (of the eye) and is often present in individuals with asthma. Rhinitis can also be caused by food allergies. Some individuals, without evidence of allergic sensitization, will have rhinitis in reaction to nonspecific irritant stimuli such as cold dry air, perfumes, paint fumes, and cigarette smoke. This condition is called vasomotor rhinitis. Severe rhinitis may result from injury to the nasal membranes such as occurs after smoke inhalation, sinusitis, or after nasal surgery.

The rhinitis that is most familiar to everyone is infectious rhinitis caused by viruses such as the common cold virus. Initially, viral rhinitis is characterized by clear, watery rhinorrhea that is accompanied by sneezing and nasal obstruction. Edema of the nasal mucosa produces occlusion of the sinus ostia, with resulting facial pain, or of the Eustachian tube, with resulting ear fullness. The nasal drainage may become cloudy due to the presence of micro-organisms and cellular debris. Responsible viruses include rhinoviruses, respiratory syncytial virus, parainfluenza, influenza and adenoviruses. Fever may accompany viral rhinitis, especially if there is bacterial superinfection by streptococcal organisms.

Rhinosinusitis, in which inflammation of the mucosa of the nasal sinuses occur together with the nasal membranes, is especially aggravating because it is accompanied by prolonged mucopurulent nasal discharge, facial pain and pressure, olfactory disturbance, and post-nasal drainage with cough. Conditions of the upper airways in which rhinitis is a component are described in detail by M.S. Dykewicz et al. (Diagnosis and management of rhinitis: Complete guidelines of the Joint Task Force on practice parameters in allergy, asthma and immunology. Annals Allergy Asthma Immunology 81: 478-518, 1998).

The time-course of the nasal response to irritants can be exactly chronicled in the laboratory. In patients with sensitivity, provocation with the allergen will result in an immediate response of severe sneezing, itching, hyper-secretion and a moderate sense of obstruction. These events peak at 30 minutes and last for about 90 minutes after provocation. The immediate response is followed by the late and/or delayed response in which severe nasal congestion and a sense of obstruction is the primary symptom. The late response to a single challenge begins 4 hours after provocation, peaks at 8 hours, and fades at 12 hours. The delayed response begins at 24 hours after provocation, peaks at 36 hours and fades at 56 hours. During the peak time of late nasal obstruction, the nostril to nasopharynx pressure gradient rises by 20 to 24 cm of water as measured by rhinomanometry, illustrating the increase in airflow resistance caused by rhinitis.

Nasal stuffiness is an important contributory factor to other breathing disorders of the upper airways, such as snoring and sleep apnea, as reviewed by M.B. Scharf and A.P.Cohen (Diagnostic and treatment implications of nasal obstruction in snoring and obstructive sleep apnea. Annals Allergy Asthma Immunology 81: 279-290, 1998). As mentioned previously, airflow through the nasal passages contributes to 50% of respiratory resistance. When airflow is impeded, there must be compensatory increased respiratory force and mouth breathing commences. While individuals with nasal congestion may be capable of breathing through the nose while awake, they must exert more effort to draw air through the nasal airway during sleep because pharyngeal muscles relax during sleep and this relaxation narrows the airway passage in the back of the mouth. The additional respiratory effort produces a greater vacuum in the throat, which pushes the throat tissues, including the tongue, towards the pharynx and further reduces the space for airflow. Mouth breathing also reduces pharyngeal airspace and contributes to snoring and the related condition known as obstructive sleep disorder.

Snoring and obstructive sleep disorders (also called obstructive sleep hypoapnea/apnea) are breathing disorders of the upper airways. The pathophysiological bases of these disorders are described in many monographs. Briefly, snoring consists of audible sounds produced during sleep caused by vibrations of throat muscles. The throat muscles vibrate more readily when they are relaxed during sleep and when air velocity is high. Obstruction of the pharynx by hypertrophied adenoids (tonsils) or obesity can also cause excessive snoring. J.M. Truelson in an eMedicine article provides an elegant description of the underlying mechanisms:
"Two basic principles of fluid flow can be applied to give some additional insight to the effects of airway narrowing - the Bernoulli principle and the Venturi effect.

The Bernoulli principle describes fluid flow in a column. A partial vacuum exists at the outer edges of a column of moving fluid such that the faster the flow, the greater will be the partial vacuum. The smaller the column, the faster the flow. An every day example of this is a paper straw. If you suck too hard on a straw, it collapses. If you suck less hard or the straw is more rigid, it does not collapse.

The Venturi effect deals with airflow accelerating as a current of air enters a narrow passageway. The wind blowing between buildings or water coming out of a hose partially occluded by the thumb are examples of this effect. Added to these effects are the distensible and moveable walls of the column of air in question-the pharynx.

Sleep apneics and normal individuals differ in their closing pressures and airway resistance due to the anatomy and the pliability of the walls of the pharynx. In a non-snoring adult the negative pressure required to close the upper airway is less than (more negative) -25 cm water. Snoring adults have a much more pliable airway, with closure during sleep occurring at pressures ranging from -2 to -10 cm water.

The cumulative effect of all these factors results in a vicious cycle, which eventuates in maximal airway closure permitted by the distensibility and relaxation of the airway. The cycle is only broken by arousal, disrupting the sleep."

Individuals with persistent snoring are not considered "normal" because snoring indicates some degree of airway obstruction. If snoring and sleep disturbances do not self-correct, the sleep disruption will cause fatigue, daytime sleepiness, short-term memory loss, decreased job effectiveness, and increased risk of motor vehicle accidents. The individual is less alert. There is also evidence of increased risk of heart attacks, strokes, high blood pressure, mood alteration, and sexual dysfunction in such individuals.

Ultimately, when airflow cannot be maintained by inspiratory force for any reason, the individual will progress to respiratory failure and the technical term describing the difficulty in breathing is "dyspnea." Dyspnea as a symptom is expressed as sensations of choking and suffocation. As a sign, it is expressed as labored breathing and inadequate ventilation with a rise in plasma carbon dioxide tension. Dyspnea occurs in serious disorders such as pneumonia, congestive heart failure, asthma, chronic obstructive pulmonary disease, emphysema, cystic fibrosis, muscular paralysis or dystrophy, Parkinson's disease, lung cancer, debilitation from wasting diseases and the like. The sense of suffocation, encompassed in dyspnea, is a frightening experience at the end of life.

Menthol, camphor and eucalyptus oil have been used since ancient times as remedies for nasal irritation and for refreshment of nasal sensations. These compounds are, however, not effective for rhinitis and may in fact exacerbate nasal congestion and obstruction, especially in the late and delayed stages of rhinitis. Sympathomimetic vasoconstrictors (decongestants) reduce nasal blood flow, but have a number of adverse side-effects, including rebound hyperemia (rhinitis medicamentosum). Disodium cromoglycate is effective for allergic rhinitis, but onset of effect is slow. By far, the most effective medications for seasonal, perennial and non-allergic rhinitis are the potent glucocorticosteroids administered as nasal sprays in manual pump-operated metered atomizers (e.g. Flonase®, Rhinocort®, Nasonex® and Nasocort®), as mouth or nasal inhalers or as nose-drops. These drugs reduce nasal membrane inflammation and the symptoms and signs of allergic rhinitis. These compounds do not, however, provide immediate sensory relief for nasal stuffiness, and are of limited efficacy for relieving the discomforts of infectious rhinitis and rhinosinusitis. Corticosteroids are effective in rhinitis associated with eosinophil-dominated inflammation (e.g. allergic rhinitis), but not in rhinitis associated with neutrophil-dominated inflammation (e.g. common cold, infectious rhinitis, sinusitis).

Spray mists and nose drops containing mixtures of herbal oils, including peppermint oil, are available as non-FDA regulated products for snoring, but no clinical evidence of efficacy for these preparations has been published in the medical literature. Mechanical devices for continuous positive airway pressure (CPAP) assisted breathing are available for severe snoring, obstructive sleep disorders, and for dyspnea from respiratory disorders. These devices are expensive and require patient cooperation.

### Delivery to Targets on Skin Surfaces and Mucous Membranes

Pharmaceutical carriers or vehicles suitable for the topical administration of icilin and combinations provided herein include any such carriers known to those skilled in the art to be suitable for the particular mode of administration. In addition, the compounds may be formulated as the sole pharmaceutically active ingredient in the composition or may be combined with other active ingredients. The effective concentration may be determined empirically by testing the compounds using in vitro and in vivo systems. Thus, preferred carriers are dermatologically acceptable vehicles well known to the art. The cold receptor agonist of this invention may be dispersed in such a vehicle as an emulsion where it is either in solid form or is solubilized and is in either the aqueous or the oil phase.

The rate of drug absorption across the skin surface is dependent on drug concentration in the formulation, its water solubility and its oil/water partition co-efficient between the stratum corneum and the formulation. The physical form of icilin to be delivered to dermal receptors is optimized by design for penetration and sufficient duration of action. Although icilin may be administered dissolved in a solvent such as 1,2-propanediol, more preferably it is suspended as a solid in a liniment (emulsion) such as an ointment or cream, or it is administered as a dry powder admixed with other solids. In the solid form, icilin may be modified by re-crystallization to a particle with maximal surface area, or it may be incorporated onto nanospheres or incorporated into nanoparticles or liposomes, as such methods are now known in the art. For icilin to reach its target in the dermis, is must cross the skin by intracellular (through cells) or intercellular (between cells) routes. Occulsive bandages that increase the degree of skin hydration and prolong the contact of the drug with the skin enhance skin absorption of drugs across an intact skin, and are contemplated for practicing this invention.

Enhancers of skin penetration may usefully be included in embodiments of the invention and include certain surfactants, a drug such as azone, alcohol, acetone, propylene glycol and polyethylene glycol of appropriate molecular weight. Suitable surfactants include a common type of anionic synthetic surfactant are the sodium or potassium alkyl benzene sulfonates, in which the alkyl group contains from about 9 to about 15 carbon atoms. Non-ionic surfactants that may enhance retention on the skin and facilitate absorption are polyoxyethylene polymers. Representative of such agents are the esters or partial esters of fatty acids containing from 6 to 22 carbon atoms, such as octanoic, palmitic, stearic, linoleic, myristic, and oleic acids with an aliphatic polyhydric alcohol or its cyclic anhydride such as, for example, ethylene glycol, glycerol, erythritol, arabitol, mannitol, sorbitol, and the polyoxyethylene and polyoxypropylene derivatives of these esters.

It is well understood in the art that the precise dosage and duration of treatment is a function of the tissue being treated and may be determined empirically using known testing protocols or by extrapolation from in vivo or in vitro test data. It is to be noted that concentrations and dosage values may also vary with the age of the individual treated. It is to be further understood that for any particular subject, specific dosage regimens should be adjusted over time according to the individual need and the professional judgment of the person administering or supervising the administration of the formulations, and that the concentration ranges set forth herein are exemplary only and are not intended to limit the scope or practice of the claimed formulations.

The Formula 1 compounds (icilin and icilin analogs) are typically included at concentrations of 0. 1% w/w up to 50% w/w or higher. Preferable concentrations are in the range of 0.5 % w/w to about 25% w/w, more preferably 1% w/w to 25% w/w, yet more preferably greater than about 1% w/w to about 10% w/w, and most preferably greater than 1% w/w up to about 5% w/w. Aqueous suspensions and formulations contain 1% w/w or more.

Broadly, then, the suitable therapeutic compositions may be formulated as a solution, suspension, emulsion or the like, and may also be formulated as creams, gels, ointments, emulsions, solutions, elixirs, lotions, suspensions, tinctures, pastes, foams, aerosols, irrigations, sprays, suppositories, bandages, or any other formulations suitable for topical administration.

The composition manufactured according to the present invention may be incorporated into an occlusive bandage containing at least one water-insoluble, pharmacologically approved, alkyl cellulose or hydroxyalkyl cellulose. Alkyl cellulose or hydroxyalkyl cellulose polymers for use in this invention include methyl cellulose, ethyl cellulose, propyl cellulose, butyl cellulose, cellulose acetate, hydroxymethyl cellulose, hydroxyethyl cellulose, hydroxypropyl cellulose, hydroxybutyl cellulose, and ethylhydroxyethyl cellulose, alone or in combination. For ethyl cellulose polymers, the preferred characteristics include an ethoxyl content between 42 and 52%, and more preferably between 44 and 50%, and for a 5% by weight of polymer in a 80/20 toluene/ethanol solution, a viscosity of between 2 and 500 cps, and more preferably between 4 and 400 cps. In addition, a plasticizer or a cross linking agent may be used to modify the polymer's characteristics. For example, esters such as dibutyl or diethyl phthalate, amides such as diethyldiphenyl urea, vegetable oils, fatty acids and alcohols such as acid oleic and myristyl may be used in combination with the cellulose derivative.

The solvent used for dissolving icilin may be a nonaqueous pharmacologically approved solvent with good penetration characteristics. Suitable organic materials useful as the solvent or a part of a solvent system are as follows: propylene glycol polyethylene glycol (M.W. 200-600), polypropylene glycol (M.W. 425-2025), glycerine, sorbitol esters, 1,2,6-hexanetriol ethanol, isopropanol, diethyl tartrate, butanediol and mixtures thereof. Such solvent systems can also contain water. Some examples include ethoxydiglycol, and 1 methyl-2 pyrrolidone, and propanediols, alone or in combination. The preferred solvent for use in this invention is a propanediol containing 0. 2 to 5% of icilin by weight.

For one example, an ointment containing icilin, 0.02 to 5% by weight, may be composed of emulsifying wax, white petrolatum and propylene glycol, butylated hydroxyanisole, propyl gallate, citric acid and lactic acid. A lotion containing icilin, 0.1 to 5% by weight, may be composed of a smooth, homogeneous, opaque emulsion composed of benzyl alcohol 2% (wt/wt) as preservative, emulsifyng wax, glycerin, isopropyl palmitate or myristate, lactic acid, purified water, and polyethylene glycol 400.

For another example, an antipruritic ointment may contain the following ingredients: a homogeneous melt of 50.0% methyl salicylate, 25.0% white beeswax, 25.0% anhydrous lanolin to which is added 2% by weight of icilin and 1% by weight of 0.5% of di-sec-butyl-n-octyl phosphine oxide. The mixture is warmed and then allowed to solidify. A soft ointment is produced having a soothing effect on the skin accompanied by a cooling effect.

Solutions and suspensions for topical administration are formulated to contain an amount of one or more compounds effective to deliver an anti-pruritic amount, typically at a concentration of between about 0.1-50% w/w, preferably at least more than 1% w/w, more preferably more than 2% w/w of one or more of the compounds provided herein. The balance is water, a suitable organic solvent or other suitable solvent or buffer.

Solutions or suspensions used for local application can include any of the following components: a sterile diluent, such as purified water , saline solution, polyethylene glycol glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity such as sodium chloride or dextrose.

Liquid preparations can be enclosed in ampoules, disposable syringes or multiple dose vials made of glass, plastic or other suitable material Suitable carriers may include physiological saline or phosphate buffered saline (PBS), and the suspensions and solutions may contain thickening and solubilizing agents, such as glucose, polyethylene glycol, and polypropylene glycol and mixtures thereof. Suitably prepared solutions and suspension may also be topically applied to the eyes and mucosa. Solutions, particularly those intended for ophthalmic use, maybe formulated as 0.01%-10% w/w isotonic solutions, pH about 5-7, with appropriate salts, and preferably containing one or more of the compounds herein at a concentration of about 0.1% w/w preferably greater than 1% w/w, up to 50% w/w or more. Suitable ophthalmic solutions are known (see, e.g. U.S. Pat. No. 5,116,868, which describes typical compositions of ophthalmic irrigation solutions and solutions for topical application). Such solutions, which have a pH adjusted to about 7.4, contain, for example, 90-100 mM sodium chloride, 4-6 mM dibasic potassium phosphate, 4-6 AM dibasic sodium phosphate, 8-12 mM sodium citrate, 0.5-1.5 mM magnesium chloride, 1.5-2.5 mM calcium chloride, 15-25 MM sodium acetate, 10-20 mM D.L.-sodium .beta.-hydroxy butyrate and 5-5.5 mM glucose.

### Delivery to Intranasal Cold Receptors

For delivery to the nose the therapeutic compositions manufactured according to this invention may be administered by nasal aerosol to deliver the active ingredient onto mucosal surfaces for topical actions. Such compositions are prepared according to techniques well-known in the art of pharmaceutical formulation and may be prepared as solutions in saline, employing benzyl alcohol, benzalkonium chloride or other suitable preservatives, absorption promoters to enhance bioavailability and bioadhesiveness for prolonged contact, and/or other solubilizing or dispersing agents known in the art. Thus, a composition for administration to the intranasal or intrabuccal surfaces is particularly contemplated that comprises a solution of icilin dissolved or dispersed in a pharmaceutically acceptable diluent (carrier). The solvent may be 1,2-propanediol, 1,3-propanediol and a variety of aqueous carriers can be used, e.g. buffered water, 0.9 percent saline, buffered aqueous-ethanol solutions and the like. These compositions can be sterilized by conventional, well-known sterilization techniques, or can be sterile filtered. The resulting solutions can be packaged for use as is or mixed as an adjuvant to another medication. A composition can contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

An example of a nasal mist to which icilin may be added is Ayr® Saline Nasal Mist, which is an isotonic saline (sodium chloride) solution, buffered with sodium phosphate, and preserved with disodium EDTA and benzalkonium chloride. An example of a nasal spray decongestant is Afrin® Severe Congestion Nasal Spray with Menthol, a product of Schering-Plough, Inc. It is used: "For the temporary relief of nasal congestion due to a cold, hay fever or other upper respiratory allergies or associated with sinusitis. Shrinks swollen nasal membranes so you can breathe freely." The active ingredients is oxymetazoline hydrochloride (0.05%), a sympathomimetic amine vasoconstrictor decongestant, and the inactive ingredients are listed as benzalkonium chloride, benzyl alcohol, camphor, edetate disodium, eucalyptol, menthol, polysorbate 80, propylene glycol, sodium phosphate dibasic, sodium phosphate monobasic, and water. Similar items for upper respiratory ailments, including the common cold, cough and bronchitis, are products of Vicks Corporation, a division of Proctor and Gamble. These products include Vapor Inhaler® (levamphetamine, as a nasal decongestant), VapoRub® containing menthol and camphor, and VapoSteam® containing camphor, a chemical similar to menthol. It is expected that icilin alone or as adjunct to like products can achieve an equal if not better treatment of nasal mucosal inflammation.

The concentration of icilin utilized is usually at or at least about 0.10 percent to as much as about 2 percent by weight and is selected primarily by fluid volumes, in accordance with the particular mode of administration selected. Thus, a typical pharmaceutical composition for delivery can be made up to contain about 0.25 mg/ml to about 150 mg/ml of the icilin. Actual methods for preparing compounds are known or apparent to those skilled in the art. For use in combination with menthol analogs, the ideal molar ratio of icilin to menthol analog is about 1 to 1, with a range of 1 to 0.5, 2 to 1, and 3 to 1.

A second form of delivery of icilin to the nasal cold receptors is to administer icilin in powder form; by itself or admixed to an inert carrier such as calcium carbonate or lactose; or in conjunction with a nasal condiment such as tobacco snuff. The icilin may be prepared in micronized form, by granulation, drying, and sizing or milling to a specified particle size and thus to have a high surface area for interaction with cold receptors. An excipient (inert substances that form a vehicle for the active ingredients) such as lactose may be used for formulation to obtain uniform consistency and a uniform drug loading.

### Delivery to Bronchial Cold Receptors

The surface of the nasal cavities and the bronchi and bronchioles contain nerve endings that generate the sensations that initiate sneezing and cough. By producing a non-irritant, non-odorous stimulation of cold receptors, the desire to sneeze and cough is diminished. For aerosol administration to portions of the upper respiratory tract, icilin is preferably supplied in solution such as aqueous propylene glycol solution along with surfactant and propellant. Typical percentages of a icilin or about 0.05 percent to about 2 percent by weight, and preferably about 0.05 percent to about 0.5 percent. The surfactant must of course, be nontoxic, and preferably soluble in the propellant. Representative of such agents are the esters or partial esters of fatty acids containing from 6 to 22 carbon atoms, such as octanoic, palmitic, stearic, linoleic, and oleic acids with an aliphatic polyhydric alcohol or its cyclic anhydride such as, for example, ethylene glycol, glycerol, erythritol, arabitol, mannitol, sorbitol, and the polyoxyethylene and polyoxypropylene derivatives of these esters. Mixed esters, such as mixed or natural glycerides can be employed. The surfactant can constitute about 0.1 to about 20 percent by weight of the composition, and preferably about 0.25 to about 5 percent. The balance of the composition is ordinarily propellant. Liquefied propellants are typically gases at ambient conditions, and are condensed under pressure. Among suitable liquefied propellants are the lower alkanes containing up to 5 carbons, such as butane and propane; or preferably fluorinated alkanes. In producing the aerosol, a container equipped with a suitable valve is filled with the appropriate propellant, containing the finely divided compounds and surfactant. The ingredients are thus maintained at an elevated pressure until released by action of the valve. A pump-activated spray using air as propellant (atomizer or nebulizer) is also contemplated.

Another method for delivery of icilin is to prepare it by spray drying with a hydrophilic excipient, e.g. povidone, lactose, and deliver it using dry power inhalers. Such methods of drug preparations for inhalation have been described by Gordon et al. (U.S. Pat. 6,365,190). The advantage of this method for icilin is that it may have a more prolonged action when administered in dry power versus in soluble forms.

### Treatment of Gastrointestinal Dysfunction

The gastrointestinal tract begins at the lips and ends at the anal verge. The gut is a complex organ with many intrinsic components of the nervous system that function to regulate motility, sensations, digestion and secretion. The walls of the digestive tract are organized into four main layers: mucosa, submucosa, muscularis externa, and serosa. The mucosa consists of an epithelium with basement membrane (called the lamina propria), loose connective tissue, blood vessels, and lymph tissues. The submucosa contains loose connective tissue, glands, nerves, and blood vessels. The nerve fibers of the submucosa form a network or plexus called the plexus of Meissner. The muscularis externa consists of two bands of smooth muscle cells, the internal layer is composed of circular smooth muscle and the external layer is composed of longitudinal fibers. Interspersed between the muscle fibers is a nerve plexus called the plexus of Auerbach.

The mucosal lining of the gut lumen consists of a single layer of epithelial cells. The epithelium of the small (ileum) and large (colon) intestine have high metabolic activity and a turnover rate of about 5 days, that is, within a period of 5 days, the entire lining is shed and renewed. This is a turnover of about 1/4 pounds of cells per day. Many gastrointestinal tract disorders are characterized by depletion of or damage to intestinal mucosa resulting from exposure to environmental agents, from inflammatory responses, and from autoimmune diseases, infections, or physical injuries, and the like. After injury to the gut, especially to the mucosa, inflammation may disrupt the enteric nervous system and contribute to disorders, such as irritable bowel disease. Key elements of dysfunction are usually the mucosa and the intrinsic nervous system. The primary manifestations of gastrointestinal dysfunction are pain, a sense of distension, decreased or increased frequency of bowel movements, intra-luminal bleeding, and flatulence.

The gut has a simplified "brain". The complex nerve network in the myenteric and submucosal plexuses has about 100 million neurons (the enteric nervous system). The efferents in the submucosal Meissner plexus regulate secretion by intestinal glands. The efferents in the myenteric Auerbach plexus control the rhythmic contraction of circular and longitudinal muscles that is called peristalsis. Visceral sensory afferent nerve endings are located throughout the submucosa and the Meissner plexus. It is known that the gut mucosa contains sensory afferents (A delta and C) fibers that code for thermal and nociceptive signals. The cell bodies of the afferents are located either in autonomic ganglia or in dorsal root ganglia. Much of the sensory information from the gut is conveyed to the brain via cranial nerves of the parasympathetic nervous system, principally the vagus. Visceral sensations of pain are, however, also transmitted via sympathetic afferent nerves into the spinal cord.
*Inflammation in the Gut, Visceral Nerves, and Treatment of Visceral Discomfort.* Various regions of the intestinal tract, including the esophagus, stomach, ileum, colon, and rectum may be injured by autoimmune disorders, by infections, by psychogenic disorders, and by chemical and physical agents. The discomfort and pain of functional dysphagia (indigestion), gastrointestinal esophageal reflux disease (GERD - also known as heartburn), gastritis (e.g. caused by concentrated alcohol drink), or peptic ulcer disease (stomach and duodenal lining erosion) are caused by stimulation of visceral pain nerve endings located in the mucosa of the esophagus, stomach and duodenum. In the lower bowel, a major functional disorder is the irritable bowel syndrome. Two important organic diseases of the lower bowel are ulcerative colitis and regional enteritis (Crohn's disease). These two organic disorders are collectively referred to as inflammatory bowel disease. Other lower bowel inflammatory conditions are diverticulitis, celiac disease, lactose intolerance, chronic pancreatitis and regional ileitis. The primary clinical manifestations of these disorders are pain, a sense of distension, decreased or increased frequency of bowel movements, intra-luminal bleeding, and flatulence.

Current treatment for inflammatory gut diseases includes antacids, histamine-R2-blocking agents, antibiotics against *Helicobacter pylori,* anti-inflammatory drugs such as glucocorticosteroids and NSAIDS, anti-spasmodic agents, anti-diarrheal drugs, and, in the case of ulcerative colitis, surgery to remove the affected tissues. In a review, Sheikh and Wright (Irritable Bowel Syndrome: Current Concepts and Future Prospects. Hospital Practice, March 1999, pg. 31-38) describe a new category of "Anti-Afferent" agents designed to reduce intestinal perception by blocking the afferent nerve receptors.

In some patients, certain aromatic oils can relax smooth muscle and relieve pain caused by cramps and gas. Peppermint oil is the most commonly used agent in this class. Enteric-coated capsules containing peppermint oil have beneficial effects on patients with the irritable bowel syndrome. Peppermint oil is 30 to 55% menthol and it is thought that menthol is the active ingredient. In double-blind placebo-controlled studies, the frequency of abdominal pain, discomfort of abdominal distension, bowel movements, borborygmi and flatulence were decreased by encapsulated peppermint oil (Colpermin®). Colpermin® is approved for sale in Europe and in the United Kingdom, but not in the United States perhaps because some of the constituents of the oil, for example, pulgeone may be toxic to the liver. It is thought that the menthol content of peppermint oil may locally diminish smooth muscle contractility as well as reduce afferent discharge of sensory nerve endings in the enteric mucosa. However, menthol is not very potent, can cause local irritation at high concentrations, and has a relatively short duration of action of about 5 to 15 minutes because it is rapidly re-distributed in tissues and is metabolized.

*Hypothesis for Mechanisms of Icilin Compounds in Treatment of Visceral Discomfort and Inflammation in the Gut.* The nerve endings in the gastrointestinal tract lining, like the nerve endings of skin and of the mucous membranes in the airways and eyes, contain receptors that recognize signals coding for temperature and for pain. I find that when icilin and related analogs are applied to the lips, delivered in chewing gum or as a tablet, enteric-coated pill or capsule, produce signals of cold and counteract signals for pain and irritation from the gastrointestinal tract. I postulate that these "anti-afferent" actions are therapeutically use for attenuating the discomforts of cheilitis, stomatitis, mucositis, esophagitis, enteritis, colitis, and proctitis. As such, the icilin compounds are useful for lip inflammation, inflammation of the mouth, esophageal dysfunction, irritable bowel disease, irritable bowel syndrome, and other forms of gastrointestinal dysfunction

Cold receptors exist on visceral nerve afferents of the lips, mouth, esophagus, ileum and colon and that these receptors are activated by icilin compounds. Without being limited to theory I believe that stimulation of cold receptors by icilin and related analogs produces sensations that counteract the pain and discomforts of gut inflammation. The major advantages of using icilin and related analogs are its properties of non-irritancy and long duration of action. For example, icilin applied as an ointment or powder to the lips can produce sensations of cold lasting for several hours and no burning sensations or harsh tastes are experienced. In addition to its effects on enteric nerve endings, icilin may also have a direct action on smooth muscle cation channels; without wishing to be bound by theory here it appears to act as an inhibitor of cation movement preventing muscle contraction and reducing the irritability of smooth muscle that occurs in inflammatory states. The sensory effects of icilin compounds and local anesthetic action on nerve endings and smooth muscles are believed to be the mechanisms for its use in treatment visceral discomfort and inflammation of the lining of the gut.

It has been shown that enteric-coated capsules containing peppermint oil have beneficial effects on patients with the irritable bowel syndrome. Peppermint oil is 30 to 55% menthol and it is thought that menthol is the active ingredient. In double-blind placebo-controlled studies, the frequency of abdominal pain, discomfort of abdominal distension, bowel movements, borborygmi and flatulence were decreased by encapsulated peppermint oil (Colpermin®). It was hypothesized that the menthol content of peppermint oil may locally diminish the afferent discharge of sensory nerve endings to evoke irritability in the enteric mucosa. This is an "anti-afferent" mechanism of action. However, peppermint oil has toxic properties and the safety of human use has been questioned. Peppermint oil is a complex mixture of substances and it contains pulegone, a recognized hepatotoxin [Nair,B., Final report on the safety assessment of Mentha Piperita (Peppermint) Oil, Mentha Piperita (Peppermint) Leaf Extract, Mentha Piperita (Peppermint) Leaf, and Mentha Piperita (Peppermint) Leaf Water. Int. J. Toxicol. 20 Suppl 3: 61-73; 2001

The inventive emodiments that avoid upper gastrointestinal absorption allow localized delivery of icilin to the colon and thus attenuate the nervous irritability of this tissue. Ideally, an enteric-coated capsule including icilin, designed to withstand the acidity and digestive juices of the stomach and the small intestine, may be used alone or in combination with another pharmaceutical agent to treat irritable bowel disease.

In the Experimental section I give one example of esophageal discomfort (heartburn) being relieved by ingestion of icilin. The characteristics signs and symptoms of functional dysphagia (indigestion), gastroesophageal reflux disease (GERD), peptic ulcer diseases and various esophagitis are retro-sternal and epigastric pain, a sense of fullness and stomach discomfort, sour or bilious regurgitation, belching and occasional hypersalivation. These effects are reduced when icilin is delivered to the upper gastrointestinal tract. For the lower gastrointestinal tract, use of icilin formulations for the treatment of irritable bowel syndrome (IBS) and irritable bowel disease (IBD) are also contemplated.

*Formulation and Delivery of Icilin Compounds to Target in the Gastrointestinal Tract.* In the working example, *infra,* icilin powder, admixed with saliva and swallowed relieved heartburn. To be effective for enteritis, colitis, and severe proctitis, however, icilin compounds should preferably be taken orally, in a chemical form designed for release on targets in the intestinal tract. Localized release of icilin molecules on the inflamed gut mucosa is preferred because if there is excessive systemic absorption of the drug en passage to the target, absorbed icilin may stimulate endogenous cold receptors in the body and cause shivering, shaking, and excessive sensations of cold.

Oral formulations of icilin designed for treatment of disorders of the upper alimentary tract can be icilin incorporated into chewing gum, lozenges, lollipops, candy, syrup, mucoadhesive polymers, mucoadhesive formulations, or rapidly dissolving powders or tablets. The goal is to attach the icilin molecule to the nerve receptors in the oral cavity and esophagus as quickly as possible. Standard formulations for gastrointestinal tract drugs are described in "Remington, the science and practice of pharmacy," Alfonso R. Gennaro, Chairman of the editorial board and editor. 20th ed. Baltimore, Md. Lippincott Williams & Wilkins, 2000, Chpt. 45 Rudnic and Schwartz, Oral Solid Dosage Forms, pg. 858-893, Chpt. 46 Porter, Coating of Pharmaceutical Dosage Forms, pg. 894-902. amd Chpt. 47 Lee and Robinson, Controlled-release drug-delivery systems., pg. 903-929, all herein incorporated by reference. For application of icilin to the oral and esophageal areas, icilin may be incorporated into a mucoadhesive polymer such as polyvinylpyrrolidone, polycarbophil or sodium alginate. The suspensions, syrups, or gels are swallowed as a liquid at room temperature and adhere to the mucous membranes of the oral cavity, esophagus, and stomach lining.

For mucoadhesive pastes to be applied to the lining of the oral cavity, esophagus and stomach one may use Orabase®. Chemically Orabase® consists of plasticized hydrocarbongel, guar gum, carboxymethylcellulose, tragacanth gum and pectin. Orabase® is an adhesive-vehicle preparation and was designed for the purpose of retaining drugs applied on the oral mucous membranes. Studies with this preparation indicate that it adheres to the mucosa for 2 hours or longer after swallowing. Other per oral mucoadhesive compounds are described in U.S. Patent 6,319,513, herein incorporated by reference, and include mucoadhesive adjuncts such as titanium dioxide, silicon dioxide and clays. Remington's Chpt.45 describes the formula for a chewable tablet consisting of: magnesium trisilicate 500 mg, aluminum hydroxide, dried gel 250 mg, mannitol 300 mg, sodium saccharin 2 mg, oil of peppermint 1 mg, magnesium stearate 10 mg, and corn starch 10 mg, into which icilin may incorporated and chewed. Methods for formulating drugs into chewing gum are also commercially available from Fertin Pharma (Denmark), using Medichew® technology. Using this method, the active agent in the gum is solubilized, and various levels release and delivery can be achieved at different parts of upper gastrointestinal tract, e.g. buccal mucosa, throat, or esophagus.

The effective amount of icilin can be locally administered to the colon of the patient by oral ingestion of a unit dosage form such as a pill, tablet or capsule, comprising an effective amount of icilin which is enterically coated so as to be released from the unit dosage form in the lower intestinal tract, e.g., in the distal ileum and in the colon of the patient. A preferred unit dose is wherein icilin is present in an amount of about 10 mg to about 30 mg per pill, tablet or capsule. Enteric coatings remain intact in the stomach, but will dissolve and release the contents of the dosage form once it reaches the region where the pH is optimal for dissolution of the coating used. The purpose of an enteric coating is to substantially delay the release of the icilin until it reaches its target site of action in the ileum or colon. Aqueous film-coating technology is employed for the enteric coating of pharmaceutical dosage forms. Delayed-released oral icilin dosage forms have the potential advantage of delivering nearly all the icilin to the ileum or colon in an easily administered form.

Thus, a useful enteric coating is one that remains intact in the low pH environment of the stomach, but readily dissolved when the optimum dissolution pH of the particular coating is reached. This can vary between pH 3 to 7.5 depending upon the chemical composition of the enteric coating, but is preferably between about pH 6.8 and pH 7.2. The thickness of the coating will depend upon the solubility characteristics of the coating material and the site to be treated. The most extensively used polymer for enteric coating is cellulose acetate phthalate (CAP). However, CAP has an optimum dissolution pH greater than 6, thus early drug release may occur. Another useful polymer is polyvinyl acetate phthalate (PVAP), which is less permeable to moisture and gastric fluid, more stable to hydrolysis and able to dissolve at a lower pH, which could also result in early release of icilin in the duodenum. Another available polymer is hydroxypropyl methylcellulose phthalate. This has similar stability to PVAP and dissociates in the same pH range. Further examples of currently used polymers are those based on methacrylic acid, e.g., methacrylic acid ester copolymers with acidic ionizable groups. Dosage forms coated with methacrylic acid polymers dissolve in the ileum at about pH 6.8, and in the terminal ileum and caecum at about pH 7.2. In general coating thicknesses of about 25 to 200 microns, and especially 75 to 150 microns, are preferred using about 3 to 25 mg, preferably 8 to 15 mg of acidic coating material per square centimer of tablet or capsule surface. The precise coating thickness will however depend upon the solubility characteristics of the material used and the site to be treated.

Suitable technologies for encapsulation and drug delivery are described by Sandborn et al. (US Pat 6,166,044), incorporated by reference. Oral formulations of icilin designed for treatment of disorders and/or their signs and/or symptoms of the lower alimentary tract include sustained release preparations, enteric-coated icilin tablets or capsules, icilin attached to a glycoside, or icilin-resin complexes. These formulations avoid upper gastrointestinal absorption and permit greater delivery of icilin to the lower bowel mucosa receptors. A sustained-release tablet of icilin, may for example, contain icilin compressed with HPMC 2208 (USP) 500 mg, carnauba wax 60 mg, and HPMC 2910 (USP) 30 mg (HPMC refers to hydroxypropylmethylcellulose). An enteric-coated capsule containing icilin, designed to withstand the acidity and digestive juices of the stomach and the small intestine, may be used alone or in combination with menthol or a menthol analog to treat irritable bowel disease. Several methods described in U.S. Patent 5,849,327, U.S. Patent 5,866,619, U.S. Patent 6,140,308 and U.S. Patent 6,166,044, all herein incorporated by reference, give procedures on how such technology can be achieved. Suitable technologies for encapsulation and drug delivery are described by Sandborn et al. (U.S. Patent 6,166,044), incorporated by reference.

In one preferred embodiment, icilin in an enteric coating is administered via oral ingestion. An effective amount of icilin can be delivered to the colon of the patient by oral ingestion of a unit dosage form such as a pill, tablet or capsule, comprising icilin which is enterically coated so as to be released from the unit dosage form in the lower intestinal tract, e.g., in the distal ileum and in the colon of the patient. A preferred unit dose is wherein icilin is present in an amount of about 10 mg to about 100 mg per pill, tablet or capsule. Enteric coatings remain intact in the stomach, but will dissolve and release the contents of the dosage form once it reaches the region where the pH is optimal for dissolution of the coating used. The purpose of an enteric coating is to substantially delay the release of the icilin until it reaches its target site of action in the ileum or colon. Aqueous film-coating technology is employed for the enteric coating of pharmaceutical dosage forms. Delayed-released oral icilin dosage forms have the potential advantage of delivering nearly all the icilin to the ileum or colon in an easily administered form.

Methods for preparing a phenol group bound to a sugar are described by Brattsand et al. U.S. Patent 6,140,308, herein incorporated by reference. As earlier described, icilin compounds are described by the general formula 1-R1-phenyl, 4-R2-phenyl substituted 1,2,3,6-tetrahydropyrimidine-2-one. R1 may be a hydroxyl function and this phenol group can be coupled (derivatized) to a sugar, having the general formula icilin-R1-sugar. Icilin-R1-sugar is inactive at the cold receptor located on sensory nerve endings because the free hydroxyl group is essential for receptor stimulation. In the colon, however, bacterial sugar-hydrolyzing enzymes, called glycosidases, can act on the icilin-R1-sugar to produce local release of icilin. Methods for the synthesis of D-glycoside conjugates of drugs were described in detail by Brattsand et al., previously incorporated by reference. The synthesis of an icilin-R1-sugar molecule permits localized delivery to the lower bowel after oral ingestion. Such an icilin-R1-sugar conjugate may be formulated as an enteric-coated tablet, capsule, or pill, to further ensure intact passage through the upper gastrointestinal tract.

Another method for discrete colonic drug delivery is described in U.S. Patent 5,866,619, previously incorporated by reference. Here, using similar chemical synthetic methods as described in U.S. Patent 6,140,308, the free hydroxyl group on icilin compounds may be attached to a saccharide-containing polymer. The chemical-matrix complex is resistant to degradation in the stomach and small intestine, but bacterial enzymatic action, by hydrolyzing the oxy-sugar bond, releases the drug in the colon. Finally, Berliner and Nacht U.S. Patent 5,849,327, herein incorporated by reference, describe the use of porous microscopic beads embedded with an active agent or pro-drug and plugged with a polysaccharide that is only chemically degradable by colon-specific bacteria The microbeads further contain a coating of an enteric material that remains intact until the dosage form reaches the colon. These formulations may confine release of bioactive icilin such that it only acts locally on inflamed colon mucosa.

It is well understood in the art that the precise dosage and duration of treatment is a function of the tissue being treated and may be determined empirically using known testing protocols or by extrapolation from in vivo or in vitro test data. It is to be noted that concentrations and dosage values may also vary with the age of the individual treated. It is to be further understood that for any particular subject, specific dosage regimens should be adjusted over time according to the individual need and the professional judgment of the person administering or supervising the administration of the formulations, and that the concentration ranges set forth herein are exemplary only and are not intended to limit the scope or practice of the claimed formulations.

In the below Experimental section, Example A describes a preparation of icilin and analogs, while Examples 1-3, 5, 8, 11, 12, 19, 20 and 22 describe various therapeutic uses and aspects of the invention with human subjects. In particular, Example 5 shows that icilin and its analogs have 1) a prolonged cooling and refreshing action on the nasal surfaces that is not accompanied by odor or irritation, 2) a decongestant action on the nasal mucosa and 3) an anti-pruritic effect **Example 6** shows that icilin can be used as an adjunct in anti-inflammatory steroidal preparations. **Example 7** illustrates important pharmacokinetic differences between icilin and menthol . The effects of mentholated gum are immediate and localized to the mouth and throat By contrast, icilin delivered as a powder admixed with saliva does not act until it reaches the lower esophagus. Further protection of the icilin by standard encapsulation technology will allow it to reach the distal portions of the gastrointestinal tract such as the lower ileum and colon where it can exert its pharmacological effects as a cold receptor agonist Example 10 illustrates that sensory receptors are present within the inner lining of the mouth, and demonstrates the utility for use of icilin in the treatment of stomatitis and canker sores. **Examples 11-13** illustrate that topical application of icilin to human lips, mouth, and the skin and mucous membranes of the anorectal region is non-irritating and elicits cooling and anesthetic sensations that provide symptomatic relief of cheilitis and inflammation of the anorectal area. Thus, local, topical application of 1,2,36-tetrahydropyrimidine-2-one derivatives in a suitable vehicle to these mucocutaneous junctures and nearby tissues relieves pain, itch and discomfort; hence these pharmaceutical formulations are useful in the treatment of inflammatory disorders of these parts of the body. **Examples 14 -17** illustrate that topical application of icilin to human sexual organs and the anus is non-irritating and elicits sensations that appear to arouse sexual activity. Under the appropriate environmental setting and the proper mind set I conclude that topical application of 1,2,36-tetrahydropyrimidine-2-one derivatives (i.e., icilin and its analogs) in a suitable dermatological vehicle to these erogenous zones will enhance libido and increase the positive reinforcement from sexual intercourse; hence these pharmaceutical formulations are useful in the treatment of sexual dysfunction. **Example 18** demonstrates that icilin delivered to the nasal vestibule has cooling actions. Furthermore, the duration of action is dependent on the particle size of the inhaled powder. **Example 21** shows that there are icilin-type receptors at the back of the throat that can be activated by delivery of the drug via a mouth spray. **Examples 18 - 22** show that icilin and its analogs applied as a powder or as a nose/mouth spray have 1) a prolonged cooling and refreshing action on the nasal and throat surfaces that is not accompanied by odor or irritation, 2) a action on the nasal mucosa that relieves the sensations of obstruction, and 3) an ability to suppress snoring and facilitate sleep, and to suppress sensations of choking and suffocation.

In summary, I believe that no investigations of icilin in humans have been reported in the scientific literature other than what is discussed or described here. I point out and exemplify the unique properties of icilin in therapeutic applications, note various differences from menthol, and describe preferred embodiments for therapeutic methods of use.

### EXPERIMENTAL

### Example A

*Chemical Synthesis of Icilin and Analogs.* The methods of chemical synthesis are as described by Podesva and Do Nascimento U.S. Pat.3,821,221. Briefly, a substituted acetophenone, e.g. 3-nitroacetophenone or 3-trifluoromethylacetophenone, readily obtainable from commercial sources such as Sigma-Aldrich, Co., is mixed with diethylamine or dimethylamine in formaldehyde and refluxed in acidic solutions. After addition of a second substituent, the Mannich reaction produces a β-amino-ketone compound which is isolated. This reagent is then reacted with potassium cyanate or sodium cyanate to produce an unstable urea intermediate that proceeds to cyclize into the tetrahydropyridimine-2-one ring, with the appropriate groups on position 1 and 4 of the 1,2,3,6-tetrahydropyrimidine-2-one ring. The precipitated product is readily collected by filtration and may be recrystallized using solvents such ethyl acetate or purified on silica gel columns. The final products are solids stable at room temperature.

### Example 1 Examples where a composition prepared in accordance with the invention is employed.

A female subject with hay fever and atopic dermatitis put on a wool turtle-neck sweater for 10 minutes. Itching and a pink colored rash developed within 30 to 45 minutes on the surface of the neck. A quarter of a milliliter of a 1,2-propanediol solution containing 20 mg/ml icilin and 20 mg/ml of WS-3, a menthol analog, was applied to the neck areas that were red and pruritic. Cooling sensations were felt at the site of drug application and the desire to scratch was diminished. These effects lasted for about 1.5 hours.

### Example 2 Examples where a composition prepared in accordance with the invention is employed.

A male subject with inflamed hemorrhoids self-administered a commercial suppository, shaped as a 1.5-inch bullet, containing 25 mg of hydrocortisone. The suppository was coated with 5 mg of icilin and 5 mg of WS-23, a menthol analog. Cooling sensations were felt within 5 minutes in the anorectal area extending to the scrotum. Relief of discomfort and itch in the anorectal area lasted for longer than 3 hours. In a similar experiment, icilin, dissolved 20 mg/ml in propylene glycol was admixed with a isotonic sodium chloride solution laxative (containing monobasic sodium phosphate and dibasic sodium phosphate salts), and 2 ml of the emulsion was instilled into the rectum. Again, cooling sensations with relief of itch and discomfort were obtained for 2 hours.

### Example 3 Examples where a composition prepared in accordance with the invention is employed.

A male subject with an abrasion on his finger (caused by friction) of about 1 square centimeter received 0.8 mg of icilin applied directly to the wound with a swab stick. The dull pain previously present at the wound site began to feel cold and the pain was lessened. The sensation of cold at the wound site was not prolonged and did not spread to a larger sensory field. These results show that icilin can penetrate abraded or inflamed skin to achieve an antinociceptive action, similar to the effect obtained by applying ice to injured tissues. Crystalline menthol applied to the same site did not elicit sensations of coldness or pain reduction.

### Example 4

A small quantity of a 10 mg/ml solution of icilin dissolved in propylene glycol was applied with a cotton swab stick to the lower eyelid of a male subject Prickling sensations of cold were experienced on the lower eyelid within 10 minutes and the eyelids felt refreshed. This effect lasted for about 45 minutes. Crystals of menthol brought to the lower eyelid of this same subject initially elicited a sense of sharp discomfort. The eyelids smarted from the harsh irritant actions of menthol. The individual blinked and the eyes were momentarily closed with a small degree of lachrymation. But, after about 1 minute, this discomfort dissipated and the cooling sensations of menthol were felt on the eyelid. The sensations were refreshing and lasted for about 15 minutes and accompanied by a minty odor.

### Example 5 Examples where a composition prepared in accordance with the invention is employed.

A male subject, suffering from hay fever (in this case, seasonal allergy to grass pollen) stopped taking antihistamines for 24 hours and inhaled via the nose 0.5 mg or 2 mg (on 2 separate occasions) of icilin in powder form. No odor was detected. After drug administration, a sense of free and unobstructed airflow in the nasal cavity was experienced for about 3 hours. In the outdoors on a hill at an ambient temperature of 18°C, the sensation of breathing after icilin was that of inhaling a refreshing sea breeze. The presence of nasal discharge (allergic rhinitis), the urge to sneeze (pruritus) and the presence of itchy eyelids were curtailed and terminated by the icilin; but returned when the cold sensations diminished after 3 hours. A similar result was derived by inhalation of 0.8 mg of the acetoxy, fluoro and trimethylfluoro form of icilin but the degree of coldness and duration of action was less than that of icilin. Under identical circumstances, the inhalation of menthol crystals also produced a cooling and refreshing sensation but was accompanied by a mint flavor and an initial burning sensation. The duration of the menthol cooling effect that was achieved with 0.8 mg of menthol was 25 to 30 minutes. No overt beneficial effects were observed on the degree of nasal discharge or the urge to sneeze. Also, the eyes watered from the stinging sensations of menthol.

### Example 6

Icilin, dissolved in propylene glycol, was added to a commercial preparation of an anti-allergy nasal spray containing fluticasone to attain a concentration of about 10 mg icilin/ml solution. When sprayed into the nasal cavity the sensory qualities of icilin, as described in Example 5, were obtained. Thus, the presence of the anti-inflammatory glucocorticoid did not interfere with the actions of icilin.

### Example 7

Two mg of icilin was spread between two wafers of a fruit-flavored chewing gum and chewed for 5 minutes by a male subject. No sensations of coolness were noted in the mouth or throat. But after 12 minutes general sensations of coolness were felt in the thoracic internal structures behind the sternum, in the epigastric area. These cooling effects lasted for about 45 minutes. By contrast, the chewing of mentholated gum or mentholated candy, produced after an initial harsh taste, strong cooling of the mouth and throat that lasted only about 10 minutes.

### Example 8 Examples where a composition prepared in accordance with the invention is employed.

A male subject had angular cheilitis that was due to seasonal allergies and dry weather. Pain was experienced upon opening the mouth to a wider angle, such as in brushing of the teeth, and in the use of dental floss to clean molars. The subject used a cotton-tipped swab stick to apply 0.3 to 0.5 cc of the 2% icilin ointment prepared as in Example A to the corners of his mouth and to the vermilion borders. Within several minutes, cooling and soothing were felt in the lip region and the pain at the corners of the mouth was attenuated. There were also punctate discharges of cold spots and the effects were felt as "sparkling". In an additional experiment, three normal subjects were tested and these effects of icilin ointment on the lip region were readily demonstrated and experienced.

### Example 9

A 65-year old male subject had recurrent episodes of cold sores. With the onset of a respiratory ailment, due probably to excess alcohol consumption and cigar smoking, he anticipated the development of cold sores. The subject used a cotton-tipped swab stick to apply 0.2 to 0.4 cc of the 2% icilin ointment to the upper portion on his lips during the "prodroma" phase of the infection and reported that itchiness was diminished. Nevertheless, after two days, red papules and small blisters appeared on the upper vermilion border with characteristic pain and itch. Application of 2% icilin ointment to the lips resulted, within two to three minutes, in tingling and cooling sensations on the lips, followed by numbness, with subjective relief of discomfort. The subject repeated icilin applications with similar relief, until the cold sores disappeared 10 days after onset.

### Example 10

A male subject used a swab-stick to applied 3 mg of powdered icilin onto his buccal mucosa. Sensations of cold were felt inside the mouth at the site of application and in adajcent tissues. The effect lasted for about 30 min. An icilin solution was then prepared using 4% icilin dissolved in propylene glycol and mixed 1:1 with sterile water to yield a 2% concentration. The solution was placed in an empty 10 ml bottle with a digital plunger and nozzle valve (Migra Spray® bottle). The aerosol spray of icilin, when applied several times into the mouth, produced the same characteristic sensations of cold and decreased sensitivity.

### Example 11 Examples where a composition prepared in accordance with the invention is employed.

An adult male subject had periodic bouts of severe skin inflammation in the groin and perineal areas. The causes may have been allergic reactions to laundry detergents or to adhesives used in underwear elastic. Each episode of inflammation was accompanied by red discoloration of the skin and intense itching. The subject applied 0.5 to 0.8 cc of the 2% icilin ointment prepared as in Example A to his inflamed skin and reported that the itch immediately ceased. He also noticed that the discoloration decreased and there has been no recurrence in the last month. The subject expressed satisfaction with use of the ointment.

### Example 12 Examples where a composition prepared in accordance with the invention is employed.

A male subject with inflamed hemorrhoids applied the 2% icilin ointment (prepared as described in Example A in the Aquaphor® ointment) to his anorectal area. The estimated volume of the ointment was about 0.3 to 0.5 cc. Cooling sensations were felt within 5 minutes after application, with the effects extending to the scrotum. Relief of pain and itch in the anorectal area lasted for longer than 2 to 3 hours. In a similar experiment, the 2% icilin ointment was coated onto a commercial suppository, shaped as a 1.5-inch bullet, containing 25 mg of hydrocortisone. The estimated amount of the ointment deposited on the suppository was about 0.3 to 0.6 cc. The suppository was inserted into the anorectal area. Again, cooling sensations with relief of pain, itch and discomfort were obtained, lasting for 2 to 3 hours. In six normal individuals without hemorrhoidal disorders, application of 0.3 to 0.6 cc of the 2% icilin ointment reliably produced cooling and soothing sensations of the anorectal area that lasted for at least two hours.

### Example 13

An adult male subject with rectal pain associated with bowel movements, and due to an acute anal fissure, used a cotton-tipped swab stick to apply 0.5 to 0.8 cc of the 2% icilin ointment to his anorectal area and then proceeded immediately to the commode. The pain that normally was associated with defecation was diminished and the subject expressed relief and satisfaction with use of the ointment.

### Example 14

A middle-aged male subject, with a significant decline in sexual energy due to ageing, used a cotton-tipped swab stick to apply about 0.15 to 0.25 cc of the 2% icilin ointment described by Example A to the head of his penis. These trials were conducted on five separate occasions over a period of three days. The dose in each application was about 3 to 5 mg of icilin. This dose was considered relatively risk-free because the median lethal dose of icilin in the albino rat was greater than 1500 mg/kg of body weight when administered intraperitoneally (Wei and Seid, 1983, supra). Within two minutes after application of the ointment to the penis, tingling and prickling sensations were felt in the corona and glans, followed by tumescence. Cooling sensations were also felt on the penis, particularly on the inner foreskin. There was a slight sensation of "wetness" on the organ. The overall sensations were pleasant and sexually arousing and an increased state of libido was maintained for about two to three hours after each application and the subject was keen to proceed to the next experiment. Application of Aquaphor® ointment without icilin did not elicit similar sensations. The individual subsequently felt invigorated by these experiences, he became more robust in his sexual activities, and his self-image improved.

### Example 15

A young adult male subject with periodic erectile dysfunction due to work-related stress used a cotton-tipped swab stick to apply 0.2 to 0.5 cc of the 2% icilin ointment to the head of his penis and then proceeded immediately to have sexual intercourse with his partner. It was reported that the penis felt more rigid and that the individual had better control of his sexual rhythm. The duration of intercourse was also extended to twice the expected normal time, the subject felt more satisfied with the experience, and looked forward to the next encounter with his partner.

### Example 16

Two male subjects, on separate occasions, used a cotton-tipped swab stick to apply 0.5 to 0.8 cc of the 2% icilin ointment to the anorectal area. Within several minutes, strong cooling sensations were felt in the region around the anal sphincter extending to the scrotum. There were also punctate discharges of cold spots, so the effects were felt as "sparkling" and "provocative." Both male subjects concluded that some individuals could construe the effects of icilin at this site as an erogenous experience.

### Example 17

A female subject used a cotton-tipped swab stick to apply 0.2 to 0.4 cc of the 2% icilin ointment to the upper portion on her labia and on the clitoral surface. Within two to three minutes, tingling and prickling sensations were felt in the clitoris accompanied by tumescence. The subject then proceeded to masturbate and reported that after icilin application orgasm could be achieved more quickly and with less effort.

### Example 18

Three normal healthy subjects volunteered to inhale via the nose 5 mg of icilin in powder form. This powder was agglomerated (available from Phoenix Pharmaceuticals, Belmont, California, USA) and had a particle size distribution of greater than 50 µm in diameter. No odor was detected. After drug administration, a sense of free and unobstructed airflow in the nasal cavity was experienced for about 5 hours, with punctate sensations of cold at discrete locations in the nose. The sensations were described as soothing, cooling and refreshing. Under identical test conditions, one subject inhaled 8 mg of icilin powder obtained from Tocris Cookson, Inc. (Ellisville, MO 63021). This powder was fluffy, easily became airborne, and had a particle size distribution of less than 50 µm in diameter. Cooling sensations were felt after inhalation, but more from the upper lip than from inside the nostrils. The cooling and soothing sensations from the nasal vestibule were of short duration after the Tocris icilin and lasted less than 30 minutes.

### Example 19 Examples where a composition prepared in accordance with the invention is employed.

A male subject, suffering from seasonal allergic rhinitis, had severe nasal congestion and obstruction, which was not relieved by taking two antihistamine Allegra® tablets. He was a habitual snorer and had episodes of abrupt awakenings in the early morning hours. This individual inhaled 20 mg (in two dose applications of 10 mg each) of icilin in powder form. The icilin powder was placed on the back of his hand and sniffed vigorously. No odor was detected. After drug administration, a sense of free and unobstructed airflow in the nasal cavity developed within 15 minutes and the effect lasted for more than 8 hours. This individual then repeated the experience 5 days later. His wife then noted that he stopped snoring and snorting during sleep for the next 3 days after icilin administration Furthermore, the individual felt refreshed and invigorated by undisturbed sleep during this time. As mentioned previously, this individual had episodes of abrupt awakening in the early morning hours with sensations of choking and making gurgling sounds that would awaken his wife. These episodes did not occur during the 3 days after icilin inhalation

### Example 20 Examples where a composition prepared in accordance with the invention is employed.

A male subject suffered from perennial rhinitis that was aggravated when his daughter's dog came to visit and stayed on his bed to sleep. The individual's nasal membranes felt stuffed up, irritated, and burning, and falling asleep was difficult because he felt that he could not breathe. Mouth breathing was not comfortable, and the individual then begin taking a benzodiazepine sedative to go to sleep. However, the sedative and the restless sleep made the individual tired the next day and he could not maintain his concentration at work. This individual was then given a 5 mg dose of iciln, placed in a small plastic tube, and instructed to inhale the powder before going to sleep. He reported that airflow in the nose was smooth and cool after icilin intake and his sense of nasal stuffiness was significantly reduced. He had a good night's sleep and felt refreshed the next day.

### Example 21

An icilin solution was prepared using 10% icilin dissolved in propylene glycol and mixed 1:5 with sterile water to yield a 2% icilin concentration. The solution was placed in an empty 10 ml bottle with a digital plunger and nozzle valve (Migra Spray® bottle). The aerosol spray of icilin, when applied several times into the back of the mouth, produced the characteristic sensations of cold.

### Example 22 Examples where a composition prepared in accordance with the invention is employed.

An icilin solution was prepared using 10% icilin dissolved in propylene glycol and mixed 1:5 with Ayr® Saline Nasal Mist to yield a 2% concentration. Ayr® Saline Nasal Mist is an isotonic saline (sodium chloride) solution, buffered with sodium phosphate, and preserved with disodium EDTA and benzalkonium chloride. The bottle of Ayr® Saline Nasal Mist had a volume of 50 ml and had a nozzle for dispensing a nasal mist The icilin-saline spray mist was applied intranasally to a subject with nasal congestion from seasonal allergic rhinitis. Sensations of coolness were experienced and the sense of nasal of obstruction was relieved.

### Example 23

A male subject spread 10 mg of icilin with a swab-stick between two sticks of a fruit-flavored chewing gum and chewed the gum for 10 minutes. No sensations of coolness were noted in the mouth or throat. But after 10 minutes general sensations of coolness were felt in the retrosternal and epigastric areas. These cooling effects lasted for about 1 hour. By contrast, the chewing of mentholated gum or mentholated candy produced, after an initial harsh taste, strong cooling of the mouth and throat that lasted only about 10 minutes. The next day the subject consumed a quick meal consisting of a large-size pepperoni-sausage pizza, washed down with coca-cola. The subject reported feelings of satiety, bloating, and severe epigastric discomfort. Consumption of 5 mentholated candies (Mentos) did not affect these symptoms. However, using the icilin-chewing gum reduced all symptoms.

### Example 24

A female subject who met the diagnostic criteria for gastroesophageal reflux disease (GERD) would, on occasions after a big meal, wake in the middle of night with severe epigastric discomfort, eructations, a sour taste in the mouth, and a sense of nausea and pain. Ingestion of Tagamet® would provide some degree of relief from pain, but the other symptoms persisted. The subject was given the icilin-chewing gum wafer and instructed on its use. She reported that using the icilin-chewing gum on two different evenings helped relieve her discomfort and allowed her to go to sleep uneventfully.

### Example 25

A 60-year old female subject who had undergone a Whipple procedure for leiomyosarcoma of the duodenum 13 years previously, frequently had flatulence and abdominal cramps after meals that were not relieved by Phazyme® (simethicone). During one such episode she drank half a cup of barley water containing 10 mg of icilin powder suspended and mixed in the barley water by stirring. She reported within 25 minutes that the sense of abdominal cramping was decreased and she felt much better.

### Example 26

The field-stimulated guinea pig ileum is a standard pharmacological bioassay for studying intestinal smooth muscle contraction. A guinea pig is anesthetized with sodium pentobarbital and a segment of the ileum is cut out and electrodes put into the muscle. The muscle is placed in an oxygenated organ bath and the muscle strip connected to a Grass instrument for stimulation and recording. The rhythmic contractions of the muscle are recorded using a transducer-polygraph. Generally, after a 30-minute stabilization period, muscle contractions of constant amplitude and frequency is observed. The addition of icilin, dissolved in propylene glycol, to this bath mixture produces a dose-dependent decrease in contractile force. Propylene glycol, the solvent, alone does not produce significant contraction.

## Claims

1. Use of a therapeutically effective amount of a cold receptor agonist having Formula **I** 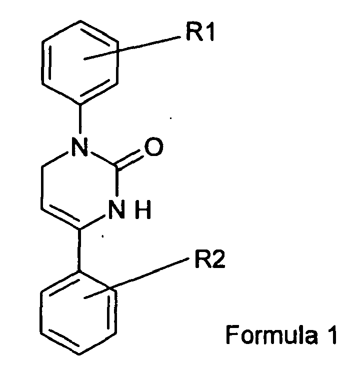 wherein R1 is hydroxy, chloro, fluoro, an alkyl of 2 to 4 carbon atoms, acetoxy, or trifluoromethyl and R2 is nitro, chloro, fluoro, an alkyl of 2 to 4 carbon atoms or trifluoromethyl
for the manufacture of a topical therapeutic composition for treating
pruritus of the skin or mucous membrane, or
sensations of pain or discomfort, in each case due to inflammation, on skin or mucous membrane.

2. Use according to claim 1 wherein the cold receptor agonist is 1-[2-hydroxyphenyl]-4-[3-nitrophenyl]-1,2,3,6-tetrahydropyrimidine-2-one.

3. Use according to claim 2 wherein
said topical therapeutic composition is for treating sensations of pain or discomfort, in each case due to inflammation, on skin or mucous membrane, and
said sensations of itch, pain or discomfort are associated with an allergy, dermatitis, rhinitis, mucositis, hemorrhoids, anal fissure, abrasion of skin, cheilitis, cold sores, or obstructed breathing.

4. Use according to claim 1, 2 or 3 wherein said treatment comprises application of the therapeutic composition to the skin or mucous membrane.

5. Use according to claim 4 wherein the cold receptor agonist is dispersed in a dermatologically acceptable vehicle.

6. Use according to claim 5 wherein the vehicle includes an oleaginous base, a mucoadhesive polymer or a mucoadhesive paste.

7. Use according to claim 5 wherein the cold receptor agonist is dispersed as an emulsion or a suspension.

8. Use according to any one of the preceding claims wherein said composition includes a pharmaceutical agent.

9. Use according to claim 5 wherein in said composition the cold receptor agonist is in an amount of from 0.01% w/w to 25% w/w.

10. Use according to claim 5 wherein the cold receptor agonist is incorporated into an occlusive bandage.

11. Use according to claim 2 wherein in said composition the cold receptor agonist is in an amount of from 0.005 wt.% to 5 wt.% and the composition further comprises one or more of a surfactant and a steroid.

12. Use according to claim 11 wherein said composition contains surfactant in an amount of 0.1 wt.% to 20 wt.% of the composition.

## Patentansprüche

1. Verwendung einer therapeutisch wirksamen Menge eines Kälterezeptoragonisten mit Formel 1, 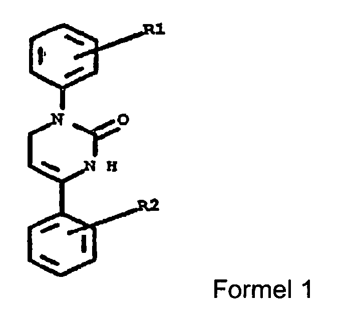 worin R1 Hydroxy, Chlor, Fluor, ein Alkyl von 2 bis 4 Kohlenstoffatomen, Acetoxy oder Trifluormethyl ist und R2 Nitro, Chlor, Fluor, ein Alkyl von 2 bis 4 Kohlenstoffatomen oder Trifluormethyl ist,
zur Herstellung einer topischen therapeutischen Zusammensetzung zur Behandlung von
Juckreiz der Haut oder der Schleimhaut oder
von Schmerzempfindungen oder Beschwerden, die in jedem Fall auf eine Entzündung zurückzuführen sind, auf der Haut oder der Schleimhaut.

2. Verwendung nach Anspruch 1, worin der Kälterezeptoragonist 1-[2-Hydroxyphenyl]-4-[3-nitrophenyl]-1,2,3,6-tetrahydropyrimidin-2-on ist.

3. Verwendung nach Anspruch 2, worin
die topische therapeutische Zusammensetzung zur Behandlung von Schmerzempfindungen oder Beschwerden, die in jedem Fall auf eine Entzündung zurückzuführen sind, auf der Haut oder der Schleimhaut gedacht ist sowie
die Empfindungen des Juckens, der Schmerzen oder der Beschwerden mit einer Allergie, Dermatitis, Rhinitis, Mucositis, Hämorrhoiden, Analfissur, Hautabschürfung, Cheilitis, Fieberblasen oder Atemobstruktion in Zusammenhang stehen.

4. Verwendung nach Anspruch 1, 2 oder 3, worin die Behandlung das Auftragen der therapeutischen Zusammensetzung auf die Haut oder Schleimhaut umfasst.

5. Verwendung nach Anspruch 4, worin der Kälterezeptoragonist in einem dermatologisch annehmbaren Vehikel dispergiert ist.

6. Verwendung nach Anspruch 5, worin das Vehikel eine ölhaltige Basis, ein mucoadhäsives Polymer oder eine mucoadhäsive Paste umfasst.

7. Verwendung nach Anspruch 5, worin der Kälterezeptoragonist als eine Emulsion oder eine Suspension dispergiert ist.

8. Verwendung nach einem der vorangegangenen Ansprüche, worin die Zusammensetzung ein pharmazeutisches Mittel umfasst.

9. Verwendung nach Anspruch 5, worin der Kälterezeptoragonist in der Zusammensetzung in einer Menge von 0,01 Gew.-% bis 25 Gew.-% vorliegt.

10. Verwendung nach Anspruch 5, worin der Kälterezeptoragonist in einen Okklusivverband inkorporiert ist.

11. Verwendung nach Anspruch 2, worin der Kälterezeptoragonist in der Zusammensetzung in einer Menge von 0,005 Gew.-% bis 5 Gew.-% vorliegt und die Zusammensetzung weiters ein oder mehrere von einem Tensid und einem Steroid umfasst.

12. Verwendung nach Anspruch 11, worin die Zusammensetzung ein Tensid in einer Menge von 0,1 Gew.-% bis 20 Gew.-% der Zusammensetzung enthält.

## Revendications

1. Utilisation d'une quantité thérapeutiquement efficace d'un agoniste d'un récepteur au froid ayant la formule I 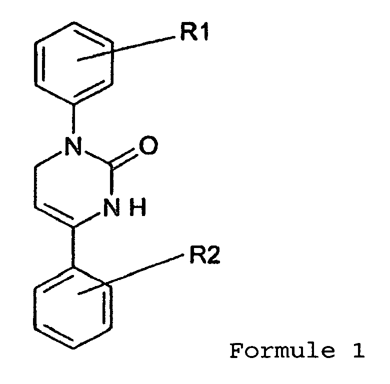 où R1 est hydroxy, chloro, fluoro, un alkyle de 2 à 4 atomes de carbone, acétoxy ou trifluorométhyle, et R2 est nitro, chloro, fluoro, un alkyle de 2 à 4 atomes de carbone ou trifluorométhyle
pour la fabrication d'une composition thérapeutique topique pour traiter
le prurite de la peau ou d'une membrane muqueuse, ou
des sensations de douleur ou de gêne, dans chaque cas dues à une inflammation, sur la peau ou la membrane muqueuse.

2. Utilisation selon la revendication 1, dans laquelle l'agoniste des récepteurs au froid est 1- [2-hydroxyphényl] - 4-[3-nitrophényl]-1,2,3,6-tétrahydropyrimidine-2-one.

3. Utilisation selon la revendication 2 dans laquelle
ladite composition thérapeutique topique est pour le traitement de sensations de douleur ou de gêne, dans chaque cas dues à une inflammation, sur la peau ou une membrane muqueuse, et
lesdites sensations de démangeaison, douleur ou gêne sont associées à une allergie, dermatite, rhinite, mucosite, hémorroïdes, fissure anale, abrasion de la peau, chéilite, herpès labial ou obstruction des voies aériennes.

4. Utilisation selon la revendication 1, 2 ou 3, dans laquelle ledit traitement comprend l'application de la composition thérapeutique à la peau ou à la membrane muqueuse.

5. Utilisation selon la revendication 4, dans laquelle l'agoniste des récepteurs au froid est dispersé dans un véhicule dermatologiquement acceptable.

6. Utilisation selon la revendication 5, dans laquelle le véhicule comprend une base oléagineuse, un polymère mucoadhésif ou une pâte mucoadhésive.

7. Utilisation selon la revendication 5, dans laquelle l'agoniste des récepteurs au froid est dispersé comme une émulsion ou une suspension.

8. Utilisation selon l'une quelconque des revendications précédentes, dans laquelle ladite composition comprend un agent pharmaceutique.

9. Utilisation selon la revendication 5, dans laquelle dans ladite composition, l'agoniste des récepteurs au froid est en une quantité de 0,01% en poids à 25% en poids.

10. Utilisation selon la revendication 5, dans laquelle l'agoniste des récepteurs au froid est incorporé dans un bandage occlusif.

11. Utilisation selon la revendication 2, dans laquelle dans ladite composition, l'agoniste des récepteurs au froid est une quantité de 0,005% en poids à 5% en poids, et la composition comprend en outre un ou plusieurs parmi un surfactant et un stéroïde.

12. Utilisation selon la revendication 11, dans laquelle ladite composition contient un surfactant en une quantité de 0,1% en poids à 20% en poids de la composition.
